# EUROPEAN PATENT SPECIFICATION

(11) **EP 1 663 145 B1**
(45) Date of publication and mention of the grant of the patent: **06.04.2011**
(21) Application number: 04785033.4
(22) Date of filing: 24.09.2004
(51) Int. Cl.: A61K 9/00, A61K 9/24, A61K 9/36

(54) **OROS-PUSH-STICK FOR CONTROLLED DELIVERY OF ACTIVE AGENTS**
OROS-PUSH-STICK FÜR DIE KONTROLLIERTE ABGABE VON WIRKSTOFFEN
BATON POUSSOIR OROS POUR L'ADMINISTRATION CONTROLEE D'AGENTS ACTIFS

(30) Priority: 26.09.2003 US 506195 P; 14.05.2004 US 571045 P
(43) Date of publication of application: 07.06.2006
(73) Proprietor: ALZA Corporation, Mountain View, CA 94039-7210 (US)
(72) Inventor: CRUZ, Evangeline, Hayward, CA 94542 (US)
(74) Representative: Williams, Paul Edwin
(86) International application number: PCT/US2004/031475
(87) International publication number: WO 2005/030166

(56) References cited:
- EP-A- 1 027 888
- WO-A-02/087512
- US-A- 4 783 337
- US-A- 4 786 503
- US-A- 4 915 954
- US-A- 5 417 682
- US-A1- 2001 031 279

## Description

### FIELD OF THE INVENTION

This invention relates generally to solid dosage forms for administering pharmaceutical agents, methods for preparing the dosage forms, and methods for providing therapeutic agents to patients in need thereof, and the like.

### BACKGROUND OF THE INVENTION

Controlled release dosage forms for delivering analgesic agents such as nonopioid analgesics and opioid analgesics are known in the art. Combination products providing delivery of relatively soluble drugs such as opioid analgesics and relatively insoluble drugs such as certain nonopioid analgesics are more difficult to prepare, however the preparation of some dosage forms has been reported. For example, U.S. Patent No. 6,245,357 discloses a dosage form to deliver an opioid analgesic such as hydromorphone or morphine in combination with a nonopioid analgesic such as acetaminophen or ibuprofen, and a pharmaceutically acceptable polymer hydrogel (maltodextrin, polyalkylene oxide, polyethylene oxide, carboxyalkylcellulose), which exhibits an osmotic pressure gradient across the bilayer interior wall and exterior wall thereby imbibing fluid into the drug compartment to form a solution or a suspension comprising the drug that is hydrodynamically and osmotically delivered through a passageway from the dosage form. This patent describes the importance of the interior wall in regulating and controlling the flow of water into the dosage form, its modulation over time as pore forming agents are eluted out of the interior wall, and its ability to compensate for loss in osmotic driving force later in the delivery period. The patent also discloses a method for administering a unit dose of opioid analgesic by administering a dose of 2 mg to 8 mg for from zero to 18 hours, and 0-2 mg for from 18-24 hours. However, the dosage forms described are suitable for and intended for once a day administration, not twice a day administration, since the dosage forms deliver opioid and nonopioid analgesics over a period of 18 to 24 hours.

High ranges of daily dosing may require drug loading in drug compositions of the dosage forms to be as much as 20% to 90% or more of the overall weight of the composition. Such loading requirements present problems in formulating compositions and fabricating dosage forms that are suitable for oral administration and can be swallowed without undue difficulty. High drug loadings present even greater problems when formulating dosage forms that are to be administered a limited number of times per day, such as for once- or twice-a-day dosing, because of the large unit dosage form required.

U.S. Patent No. 4,915,949 to Wong describes a dispenser for delivering a beneficial agent to an environment of use that includes a semipermeable wall containing a layer of expandable material that pushes a drug layer out of the compartment formed by the wall. The drug layer contains discrete tiny pills dispersed in a carrier. The exit orifice in the device is substantially the same diameter as the inner diameter of the compartment formed by the wall. U.S. patent No. 5,417, 682 to Wong describes a delivery device having a first immediate and prolonged delivery of active agent and a second initially delayed pulse delivery of another active agent.

Various devices and methods have been described having intended utility with respect to applications with high drug loading. For example, U.S. Patent Nos. 4,892,778 and 4,940,465 to Theeuwes describe dispensers for delivering a beneficial agent to an environment of use that include a semipermeable wall defining a compartment containing a layer of expandable material that pushes a drug layer out of the compartment formed by the wall. The exit orifice in the device is substantially the same diameter as the inner diameter of the compartment formed by the wall.

U.S. Patent No. 5,126,142 to Ayer describes a device for delivering an ionophore to livestock that includes a semipermeable housing in which a composition containing the ionophore and a carrier and an expandable hydrophilic layer is located, along with an additional element that imparts sufficient density to the device to retain it in the rumen-reticular sac of a ruminant animal. The ionophore and carrier are present in a dry state during storage and the composition changes to a dispensable, fluid-like state when it is in contact with the fluid environment of use. A number of different exit arrangements are described, including a plurality of holes in the end of the device and a single exit of varying diameter to control the amount of drug released per unit time due to diffusion and osmotic pumping.

Other devices in which the drug composition is delivered as a slurry, suspension or solution from a small exit orifice by the action of an expandable layer are described in U.S. Patent Nos. 5,660,861, 5,633,011; 5,190,765; 5,252,338; 5,620,705; 4,931,285; 5,006,346; 5,024,842; and 5,160,743. Typical devices include an expandable push layer and a drug layer surrounded by a semipermeable membrane. In certain instances, the drug layer is provided with a subcoat to protect the drug composition in those portions of the gastrointestinal tract having acidic pH, to delay release of the drug composition to the environment of use or to form an annealed coating in conjunction with the semipermeable membrane. However, such devices generally are not well suited as dosage forms for high drug loading due to size requirements necessary to accommodate large amounts of drug in a slurry, suspension or solution, and the need to have an oral dosage form conveniently sized so that it can be swallowed.

In the case of high drug loading, it is often preferable that a large orifice, from about 50%-100% of the inner diameter of the drug compartment, is provided in the dispensing device so that the drug layer can be dispensed in a non-fluid state. When exposed to the environment of use, drug is released from the drug layer by erosion and diffusion.

Additional U.S. patents describe formulations containing ibuprofen. U.S. Patent No. 5,021,053 describes an osmotic device for the controlled delivery of a beneficial agent to an oral cavity. The beneficial agents include ibuprofen. U.S. Patent No. 6,284,274 describes ibuprofen and other non-opiate analgesics in a bilayer tablet where the analgesic is formulated in a drug layer including PEO and PVP with a nonionic surfactant. U.S. Patent No. 4,783,337 describes an osmotic device for delivering a beneficial agent at a controlled rate, such as ibuprofen. U.S. Patent No. 4,786,503 describes a bilaminate composition of HPC in one layer and HPMC in the second layer, where ibuprofen is present in both layers. The examples disclose release rates over a period of time of 11 hours, 12 hours and 24 hours.

Accordingly, there is a need in the art for novel methods and dosage forms for drug delivery that provide control over the amount of drug delivered by immediate release as well as sustained release over a prolonged period of time, including control over the amount of drug released in each portion of the immediate release and sustained release and the rates and delivery profiles provided in each mode of release.

### SUMMARY OF THE INVENTION

Accordingly, it is a primary object of the invention to address the aforementioned need in the art by providing novel methods and dosage forms for drug delivery using sustained release dosage forms providing a burst release mechanism as well as a sustained release over a prolonged period of time. According to the present invention there is provided a sustained release dosage form for delivering a pharmaceutically active agent to a patient in need thereof, comprising:
1) a semipermeable wall defining a cavity and including an exit orifice formed or formable, therein, and
2) an erodible solid contained within the cavity and located adjacent to the exit orifice, said erodible solid comprising a binder, a disintegrant and between 70-90 weight percent of a pharmaceutically active agent,
   wherein the erodible solid is released from the dosage form through the exit orifice to provide an immediate release and a sustained release of the pharmaceutically active agent.
   In certain embodiments, the sustained release component provides an ascending rate of release of the active agent. In yet other embodiments, the sustained release component provides a zero order rate of release of the active agent.

The sustained release dosage forms are particularly useful for use with pharmaceutically active agents having a low solubility in water. In certain embodiments, the pharmaceutically active agent has a solubility in water of less than 10 mg/ml at 25°C. A class of preferred active agents includes the nonsteroidal anti-inflammatory agents, but also includes additional active agents that may be combined with these active agents, and other active agents, such as antibiotics or antiepileptics, for example.

The pharmaceutically active agent is present in the erodible solid at a high drug loading, in particular from 70%to 90% by weight, e.g. from 75% to 85% by weight.

The erodible solid comprises a disintegrant and a binding agent. The erodible solid can also optionally comprise a surfactant and an osmagent, In additional embodiments, the pharmaceutically active agent is released in an amount from the immediate release component that is controlled by the relative proportions of the disintegrant, binding agent, osmagent and solubility of the pharmaceutically active agent.

In a preferred aspect, a sustained release dosage form is provided for delivering a nonsteroidal anti-inflammatory agent to a patient in need thereof, comprising: 1) a sustained release component and 2) an immediate release component, wherein the immediate release component is not an immediate release drug coating. The sustained release component and the immediate release component are adapted to release as an credible solid. In another aspect, both the sustained release component and the immediate release component are provided in a single mechanism.

In particular embodiments, the sustained releases component provides an ascending rate of release of the nonsteroidal anti-inflammatory agent. In additional embodiments, the sustained release component provides zero order rate of release of the nonsteroidal anti-inflammatory agent.

In preferred embodiments, the nonsteroidal anti-inflammatory agent is an aryl propionic acid or a COX-2 inhibitor. Preferably, the aryl propionic acid is benoxaprofen, decibuprofen, flurbiprofen, fenoprofen, ibuprofen, indoprofen, ketoprofen, naproxen, naproxol, or oxaprozin, derivatives thereof, or mixtures thereof, and an exemplary nonsteroidal anti-inflammatory agent is ibuprofen.

The erodible solid comprises a disintegrant and a binding agent. The erodible solid can also optionally comprise a surfactant an osmagent. In additional embodiments, the nonsteroidal anti-inflammatory agent is released in an amount from the immediate release component that is controlled by the relative proportions of the disintegrant, binding agent, osmagent and solubility of the pharmaceutically active agent. In preferred embodiments, the erodible solid comprises a disintegrant, a binding agent and optionally a surfactant and an osmagent. In additional preferred embodiments, the binding agent is a hydroxyalkylcellulose, a hydroxyalkylalkylcellulose, or a polyvinylpyrrolidone. In other embodiments, the osmagent is a low molecular weight sugar such as sorbitol or mannitol, or a salt. Preferably, the osmagent, if present, is present in the erodible solid at a weight percent of from 2% to 10%. Preferably, the disintegrant is present in an amount of from 1% to 10% by weight. Preferred disintegrants include croscarmellose sodium, crospovidone, or sodium alginate, and the like. In additional embodiments, the erodible solid further comprises a nonionic or ionic surfactant. Preferably, the surfactant is present in 0.1% to 10% percent by weight in the erodible solid. Preferred nonionic surfactants include poloxamers, or a fatty acid esters of polyoxyethylene, or mixtures thereof. Preferred ionic surfactants include alkali salts of C₈-C₁₈ alkyl sulfates. In exemplary embodiments, the credible solid comprises from 1% to 10% by weight of a hydroxyalkylcellulose such as hydroxypropylcellulose, from 2% to 6% by weight of a disintegrant such as croscarmellose sodium, and 2% to 3% by weight of an ionic surfactant such as sodium lauryl sulfate. In additional embodiments, the erodible solid comprise from 1% to 10% by weight of a polyvinylpyrrolidone, from 2% to 6% by weight of a disintegrant such as croscarmellose sodium, and from 2% to 3% by weight of an ionic surfactant such as sodium lauryl sulfate.

In yet other embodiments, sustained release dosage forms are provided comprising a pharmaceutically active agent and pharmaceutically acceptable salts thereof and adapted to release as an erodible solid over a prolonged period of time, wherein the dosage form provides a burst release of the pharmaceutically active agent without the presence of a drug coating. Preferably, the dosage form provides a burst release of from
10% to 50% of the pharmaceutically active agent in the first hour after oral administration of the dosage from, or from 15% to 30% released in the first hour after oral administration. In particular embodiments, the pharmaceutically active agent has a low solubility in water, and in certain embodiments, the pharmaceutically active agent has a solubility in water of less than 10 mg/ml at 25°C.

The pharmaceutically active agent is present in the erodible solid at a high drug loading from 70% to 90% by weight. In other embodiments, the drug loading is from 75% to 85% by weight.

The erodible solid comprises a disintegrant and a binding agent. In additional embodiments, the erodible solid further optionally comprises a surfactant and/or an osmagent. The burst release can be controlled by the relative proportions of The disintegrant, binding agent, osmagent and solubility of the pharmaceutically active agent. In another aspect, the rate of release of the pharmaceutically active agent can be modulated by the presence of an osmagent in the erodible Solid.

In additional embodiments, sustained release dosage forms are provided comprising a pharmaceutically active agent and pharmaceutically acceptable salts thereof and adapted to release as an erodible solid over prolonged period of time. The rate of release of the pharmaceutically active agent can be modulated by the presence of an osmagent in the erodible solid.

In yet other embodiments, sustained release dosage forms are provided comprising a pharmaceutically active agent and pharmaceutically acceptable salts thereof, and are adapted to release as an erodible solid over a prolonged period oftime. The rate of release of the pharmaceutically active agent in the first hour can be controlled by the amount of osmagent, binding agent and disintegrant present in the erodible solid.

In additional embodiments, sustained release dosage forms are provided comprising a pharmaceutically active agent and pharmaceutically acceptable salts thereof and adapted to release as an erodible solid over a prolonged period of time. The rate of release of the pharmaceutically active agent in the first hour can be controlled by the relative rates of hydration of the osmagent, binding agent and disintegrant present in the erodible solid.

In preferred, embodiments, the pharmaceutically active agent is a nonsteroidal anti-inflammatory agent. In particular embodiments, the dosage form provides an immediate release of from 10%to 50% of the nonsteroidal anti-inflammatory agent in the first hour after oral administration of the dosage from, or from 15%to
30% released in the first hour after oral administration. In other particular embodiments, the dosage form provides an immediate release of from 15% to 30% of the nonsteroidal anti-inflammatory agent in the first hour after oral administration of the dosage form. The erodible solid comprises a disintegrant and a binding agent. The erodible solid can also comprise a surfactant and an osmagent. The surfactant can be a nonionic or ionic surfactant. Preferably, the ionic surfactant is an alkali salt of a C₈-C₁₈ alkyl sulfate, and the nonionic surfactant is a poloxamer, or a fatty acid ester of polyoxyethylene, or mixtures thereof.

In particular embodiments, the dosage from provides a zero order release from about I hour to about 10 hrs after administration. Preferably, the dosage from releases about 90% of the active agent in less than about 12 hrs. In particular embodiments, the dosage form provides a zero order rate of release for at least a portion of the delivery period. In other embodiments, the dosage form provides an ascending rate of release for at least a portion of the delivery period. Preferably, the dosage form provides a faster initial rate of release followed by a zero order rate of release of the remaining active agent. In other preferred embodiments, the dosage form provides a slow initial rate of release followed by an ascending rate of release of the remaining active agent. In yet other embodiments, the dosage form provides a fast initial rate of release followed by a slower rate of release and an ascending rate of release of the remaining active agent.

Sustained release dosage forms are also disclosed wherein the dosage form comprises: (1) a semipermeable wall defining a cavity and including an exit orifice formed or formable therein; (2) a drug layer comprising a therapeutically effective amount of an active agent such as a nonsteroidal anti-inflammatory agent contained within the cavity and located adjacent to the exit orifice; (3) a push displacement layer contained within the cavity and located distal from the exit orifice; (4) a flow-promoting layer between the inner surface of the semipermeable wall and at least the external surface of the drug layer that is opposite the wall; wherein the dosage form provides an *in vitro* rate of release of the active agent, preferably a nonsteroidal anti-inflammatory agent, for up to about 12 hours after being contacted with water in the environment of use. In preferred embodiments, the dosage form further provides an immediate release of the nonsteroidal anti-inflammatory agent without the presence of an immediate release drug coating. In particular embodiments, the dosage form provides an immediate release of from 10% to 50% of the nonsteroidal anti-inflammatory agent in the first hour after oral administration of the dosage form. In additional embodiments, the dosage form provides an immediate release of from 15% to 30% of the nonsteroidal anti-inflammatory agent in the first hour after oral administration of the dosage form.

Methods are also disclosed for providing a sustained release of a pharmaceutically active agent, comprising orally administering to a patient in need thereof at least one embodiment of the sustained release dosage forms described herein. Methods are also disclosed for providing an effective dose of a nonsteroidal anti-inflammatory agent to a patient in need thereof for an extended period of time, comprising orally administering to a patient in need thereof a sustained release dosage form described herein. Methods are also disclosed for controlling the amount and rates or release of a pharmaceutically active agent released from a sustained release dosage form in an immediate release mode and in a sustained release mode.

The sustained release dosage forms can also be used for administering additional pharmaceutically active agents such as opioid analgesics in combination with other active agents. In a preferred embodiment, a nonopioid analgesic is combined with an opioid analgesic in a sustained release dosage form, and release of both agents can be provided at rates proportional to their respective amounts in the dosage form.

Additional objects, advantages and novel features of the invention will be set forth in part in the description which follows, and in part will become apparent to those skilled in the art upon examination of the following, or may be leaned by practice of the invention.

### BRIEF DESCRIPTION OF THE DRAWINGS

FIG. 1 shows a schematic illustration of one embodiment of a dosage form according to the invention.

FIG. 2 illustrates a release profile of the active agent ibuprofen from a representative dosage form.

FIG. 3 illustrates a release profile of the active agent ibuprofen from a representative dosage form.

FIG. 4 illustrates a release profile of the active agent ibuprofen from a representative dosage form.

FIG. 5 illustrates a release profile of the active agent ibuprofen from a representative dosage form.

FIGS. 6A and B illustrate a release profile and cumulative release profile of the active agent ibuprofen from a representative dosage form.

FIG. 7 illustrates a release profile of the active agent ibuprofen from a representative dosage form.

FIG. 8 illustrates a release profile of the active agent ibuprofen from a representative dosage form.

FIG. 9 illustrates a release profile of the active agent ibuprofen from a representative dosage form.

### DETAILED DESCRIPTION OF THE INVENTION

Definitions and overview

Before the present invention is described in detail, it is to be understood that unless otherwise indicated this invention is not limited to specific pharmaceutical agents, excipients, polymers, salts, or the like, as such may vary. It is also to be understood that the terminology used herein is for the purpose of describing particular embodiments only and is not intended to limit the scope of the present invention.

It must be noted that as used herein and in the claims, the singular forms "a," "and" and "the" include plural referents unless the context clearly dictates otherwise. Thus, for example, reference to "a carrier" includes two or more carriers; reference to "a pharmaceutical agent" includes two or more pharmaceutical agents, and so forth.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

For clarity and convenience herein, the convention is utilized of designating the time of drug administration or initiation of dissolution testing as zero hours (t = 0 hours) and times following administration in appropriate time units, e.g., t = 30 minutes or t = 2 hours, etc.

As used herein, the phrase "ascending plasma profile" refers to an increase in the amount of a particular drug in the plasma of a patient over at least two sequential time intervals relative to the amount of the drug present in the plasma of the patient over the immediately preceding time interval. Generally, an ascending plasma profile will increase by at least about 10% over the time intervals exhibiting an ascending profile.

As used herein, the phrase "ascending release rate" refers to a dissolution rate that generally increases over time, such that the drug dissolves in the fluid at the environment of use at a rate that generally increases with time, rather than remaining constant or decreasing, until the dosage form is depleted of about 80% of the drug.

The terms "deliver" and "delivery" refer to separation of the pharmaceutical agent from the dosage form, wherein the pharmaceutical agent is able to dissolve in the fluid of the environment of use.

By "dosage form" is meant a pharmaceutical composition or device comprising an active pharmaceutical agent, or a pharmaceutically acceptable acid addition salt thereof, the composition or device optionally containing pharmacologically inactive ingredients, i.e., pharmaceutically acceptable excipients such as polymers, suspending agents, surfactants, disintegrants, dissolution modulating components, binders, diluents, lubricants, stabilizers, antioxidants, osmotic agents, colorants, plasticizers, coatings and the like, that are used to manufacture and deliver active pharmaceutical agents.

As used herein, the term "immediate-release" refers to the substantially complete release of at least a portion of a drug contained within a dosage form within a short time period following administration of the dosage form or initiation of dissolution testing, i.e., generally within a few minutes to about 1 hour.

As used herein, unless further specified, the term "a patient" means an individual patient and/or a population of patients in need of treatment for a disease or disorder. The patient can be any animal, typically the patient is a mammal, and preferably is a human.

By "pharmaceutically active agent," "drug," "active agent," or "compound," which are used interchangeably herein, is meant any agent, drug, compound or composition of matter, or mixture thereof, which provides some physiological, psychological, biological, or pharmacological, and often beneficial, effect when administered to a human or animal patient.

By "pharmaceutically acceptable acid addition salt" or "pharmaceutically acceptable salt," which are used interchangeably herein, are meant those salts in which the anion does not contribute significantly to the toxicity or pharmacological activity of the salt, and, as such, they are the pharmacological equivalent of the base form of the active agent. Examples of pharmaceutically acceptable acids that are useful for the purposes of salt formation include, but are not limited to, hydrochloric, hydrobromic, hydroiodic, sulfuric, citric, tartaric, methanesulfonic, fumaric, malic, maleic and mandelic acids. Pharmaceutically acceptable salts further include mucate, N-oxide, sulfate, acetate, phosphate dibasic, phosphate monobasic, acetate trihydrate, bi(heptafluorobutyrate), bi(methylcarbamate), bi(pentafluoropropionate), bi(pyridine-3-carboxylate), bi(trifluoroacetate), bitartrate, chlorhydrate, and sulfate pentahydrate, benzenesulfonate, benzoate, bicarbonate, bitartrate, bromide, calcium edetate, camsylate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methyinitrate, methylsulfate, mucate, napsylate, nitrate, pamoate (embonate), pantothenate, phosphate/diphosphate, polygalacturonate, salicylate, stearate, subacetate, succinate, sulfate, tannate, tartrate, teoclate, triethiodide, benzathine, chloroprocaine, choline, diethanolamine, ethylenediamine, meglumine, and procaine, aluminum, calcium, lithium, magnesium, potassium, sodium propionate, zinc, and the like.

As used herein, the term "proportional" (when referring to the release rate or delivery of the nonopioid analgesic and opioid analgesic from the dosage form) refers to the release or the rate of release of the two analgesic agents relative to each other, wherein the amount released is normalized to the total amount of each analgesic in the dosage form, i.e., the amount released is expressed as a percent of the total amount of each analgesic present in the dosage form. Generally, a proportional release rate of the nonopioid analgesic or of the opioid analgesic from the dosage form means that the relative release rate (expressed as percent release) or amount released (expressed as the cumulative release as a percent of the total amount present in the dosage form) of each drug is within about 20%, more preferably within about 10%, and most preferably within about 5% of the release rate or amount released of the other drug. In other words, at any point in time, the release rate of one agent (stated as a percentage of its total mount present in the dosage form) does not deviate more than about 20%, more preferably not more than about 10%, and most preferably not more than about 5% of the release rate of the other agent at the same point in time.

A drug "release rate" refers to the quantity of drug released from a dosage form per unit time, e.g., milligrams of drug released per hour (mg/hr). Drug release rates for drug dosage forms are typically measured as an *in vitro* rate of dissolution, i.e., a quantity of drug released from the dosage form per unit time measured under appropriate conditions and in a suitable fluid. For example, dissolution tests can be performed on dosage forms placed in metal coil sample holders attached to a USP Type VII bath indexer and immersed in about 50 ml of acidified water (pH = 3) equilibrated in a constant temperature water bath at 37° C. Aliquots of the release rate solutions are tested to determine the amount of drug released from the dosage form, for example, the drug can be assayed or injected into a chromatographic system to quantify the amounts of drug released during the testing intervals.

Unless otherwise specified, a drug release rate obtained at a specified time following administration refers to the *in vitro* drug release rate obtained at the specified time following implementation of an appropriate dissolution test. The time at which a specified percentage of the drug within a dosage form has been released may be referenced as the "Tₓ" value, where "x" is the percent of drug that has been released. For example, a commonly used reference measurement for evaluating drug release from dosage forms is the time at which 90% of drug within the dosage form has been released. This measurement is referred to as the "T₉₀" for the dosage form.

As used herein, the term "sustained release" refers to the release of the drug from the dosage form over a period of many hours. Generally the sustained release occurs at such a rate that blood (e.g., plasma) concentrations in the patient administered the dosage form are maintained within the therapeutic range, that is, above the minimum effective analgesic concentration or "MEAC" but below toxic levels, over a period of time of about 12 hours.

As used herein, the phrase "zero order plasma profile" refers to a substantially flat or unchanging amount of a particular drug in the plasma of a patient over a particular time interval. Generally, a zero order plasma profile will vary by no more than about 30% and preferably by no more than about 10% from one time interval to the subsequent time interval.

As used herein, the phrase "zero order release rate" refers to a substantially constant release rate, such that the drug dissolves in the fluid at the environment of use at a substantially constant rate. A zero order release rate can vary by as much as about 30% and preferably by no more than about 10% from the average release rate.

One skilled in the art will understand that effective treatment of a disease or disorder will vary according to many factors, including individual patient variability, health status such as renal and hepatic sufficiency, physical activity, and nature and relative intensity of the disease or symptoms.

The present inventors have made the surprising discovery that sustained release dosage forms can provide an immediate release of pharmaceutically active agents in the absence of an immediate release drug coating. The present inventors have further surprisingly discovered that the release rates of sustained release dosage forms can be modulated by the addition of osmagents to achieve heretofore unseen results.

Without wishing to be bound by theory, it is believed that the mechanism for this surprising discovery is related to the competition of the components of the dosage form for water, when the dosage form is placed in the presence of water in the environment of use. It is theorized that the presence of slowly hydrating components within the drug containing portion of the dosage form along with the fast hydrating disintegrant allows for the relatively rapid and preferential hydration of the disintegrant, resulting in a rapid expansion and disintegration of the drug containing portion of the dosage form in the initial stages of hydration of the dosage form, immediately after oral ingestion or initiation of dissolution testing. The presence of an osmagent in the drug containing portion of the dosage form acts to modulate the fast hydration of the disintegrants, allowing for a slowing of the release rate relative to the release rate observed in the absence of the osmagent. The substitution of faster hydrating binding agents for slower hydrating binding agents also results in a slowing of the release rate so that the release rate from the dosage form resembles the release rate from a dosage form that provides sustained release of drug without a burst release. The competition of excipients and active agents for water results in a means for controlling the release rate of the drug both in the initial stages and later during the dosing interval, providing for sustained release.

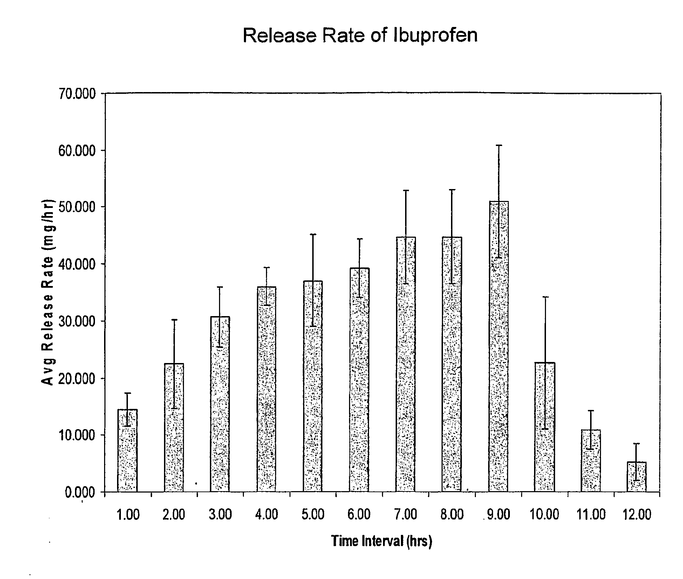

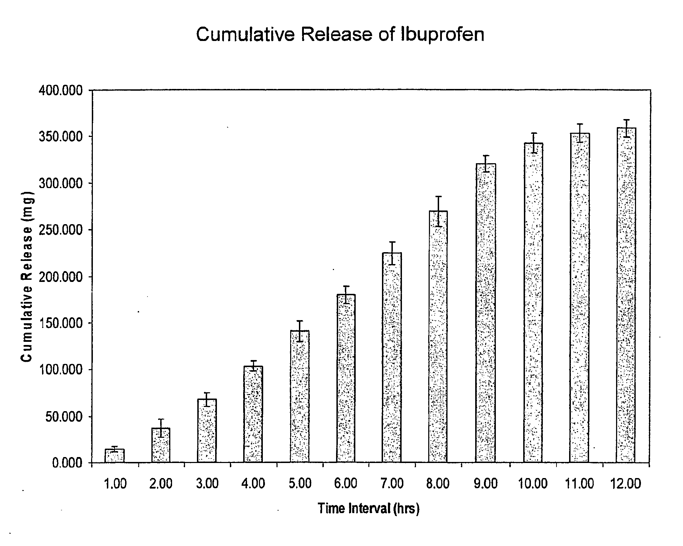

Further, the disclosed formulations can provide a high loading of a relatively insoluble active agent and further provide possible synergistic or therapeutic combinations with additional active agents, having a similar or quite different solubility. The dosage forms can exhibit proportional delivery of additional active agents (e.g., hydrocodone and ibuprofen) even though the physical properties of the active agents (e.g., their solubilities), differ markedly from each other. The formulations can be administered to a human patient in a manner to provide effective concentrations of active agents relatively quickly and to further provide a sustained release to maintain levels of active agents sufficient to treat the condition or disorder for up to about 12 or more hours. In addition, the formulations provide for substantially complete delivery of the active agent over the sustained release period. For example, Figures 6B and 7B show that essentially complete release of the active agent occurred over the period of dissolution testing.

Accordingly, sustained release dosage forms are provided for delivering a pharmaceutically active agent as an erodible solid. In a preferred embodiment, a sustained release component and immediate release component are provided in a single mechanism. In certain embodiments, the sustained release component provides an ascending rate of release of the active agent. In yet other embodiments, the sustained release component provides a zero order rate of release of the active agent.

The sustained release dosage forms are particularly useful for use with pharmaceutically active agents having a low solubility in water. In certain embodiments, the pharmaceutically active agent has a solubility in water of less than 10 mg/ml at 25°C. A class of preferred active agents includes the nonsteroidal anti-inflammatory agents, but also includes additional active agents that may be combined with these active agents, and other active agents, such as antibiotics or antiepileptics, for example.

The pharmaceutically active agent is present in the erodible solid at a high drug loading from 70% to 90% by weight, e.g. from 75% to 85% by weight.

The erodible solid comprises a disintegrant and a binding agent. The erodible solid can also optionally comprise a surfactant and an osmagent. In additional embodiments, the pharmaceutically active agent is released in an amount from the immediate release component that is controlled by the relative proportions of the disintegrant, binding agent, osmagent and solubility of the pharmaceutically active agent.

In a preferred aspect, a sustained release dosage form is provided for delivering a nonsteroidal anti-inflammatory agent to a patient in need thereof, comprising: 1) a sustained release component and 2) an immediate release components, wherein the immediate release component is not an immediate release drug coating. In preferred embodiments, both the sustained release component and the immediate release component are adapted to release as an erodible solid. In another aspect, both the sustained release component and the immediate release component are provided in a single mechanism.

In particular embodiments, the sustained release component provides an ascending rate of release of the nonsteroidal anti-inflammatory agent. In additional embodiments, the sustained release component provides zero order rate of release of the nonsteroidal anti-inflammatory agent.

In preferred embodiments, the nonsteroidal anti-inflammatory agent is an aryl propionic acid or a COX-2 inhibitor. Preferably, the aryl propionic acid is benoxaprofen, decibuprofen, flurbiprofen, fenoprofen, ibuprofen, indoprofen, ketoprofen, naproxen, naproxol, or oxaprozin, derivatives thereof, or mixtures thereof, and an exemplary nonsteroidal anti-inflammatory agent is ibuprofen.

The erodible solid comprises a disintegrant and a binding agent. The erodible solid can also optionally comprise a surfactant and an osmagent. In additional embodiments, the nonsteroidal anti-inflammatory agent is released in an amount from the immediate release component that is controlled by the relative proportions of the disintegrant, binding agent, osmagent and solubility of the pharmaceutically active agent. In preferred embodiments, the erodible solid comprises a disintegrant, a binding agent and optionally a surfactant and an osmagent. In additional preferred embodiments, the binding agent is a hydroxyalkylcellulose, a hydroxyalkylalkylcellulose, or a polyvinylpyrrolidone. In other embodiments, the osmagent is a low molecular weight sugar such as sorbitol or mannitol, or a salt. Preferably, the osmagent, if present, is present in the erodible solid at a weight percent of from 2% to 10%. Preferably, the disintegrant is present in an amount of from 1% to 10% by weight. Preferred disintegrants include croscarmellose sodium, crospovidone, or sodium alginate, and the like. In additional embodiments, the erodible solid further comprises a nonionic or ionic surfactant. Preferably, the surfactant is present in 0.1% to 10% percent by weight in the erodible solid. Preferred nonionic surfactants include poloxamers, or a fatty acid esters of polyoxyethylene, or mixtures thereof. Preferred ionic surfactants include alkali salts of C₈-C₁₈ alkyl sulfates. In exemplary embodiments, the erodible solid comprises from
1% to 10% by weight of a hydroxyalkylcellulose such as hydroxypropylcellulose, from 2% to 6% by weight of a disintegrant such as croscarmellose sodium, and 2% to 3% by weight of an ionic surfactant such as sodium lauryl sulfate. In additional embodiments, the erodible solid composes from 1% to 10% by weight of a polyvinylpyrrolidone, from 2% to 6% by weight of a disintegrant such as croscarmellose sodium, and from 2% to 3% by weight of an ionic surfactant such as sodium lauryl sulfate.

In yet other embodiments, sustained release dosage forms are provided comprising a pharmaceutically active agent and pharmaceutically acceptable salts thereof and adapted to release as an erodible solid over a prolonged period of time, wherein the dosage form provides a burst release of the pharmaceutically active agent without the presence of a drug coating. Preferably, the dosage form provides a burst release of from 10% to 50% of the pharmaceutically active agent in the first hour after oral administration of the dosage form, or from 15% to 30% released in the first hour alter oral administration. In particular embodiments, the pharmaceutically active agent has a low solubility in water, and in certain embodiments, the pharmaceutically active agent has a solubility in water of less than 10 mg/ml at 25°C.

The pharmaceutically active agent is present in the erodible solid at a high drug loading from 70% to 90% by weight. In other embodiments, the drug loading is from 75% to 85% by weight.

The erodible solid comprises a disintegrant and a binding agent. In additional embodiments, the erodible solid further optionally comprises a surfactant and/or an osmagent The burst release can be controlled by the relative proportions of the disintegrant, binding agent, osmagent and solubility of the pharmaceutically active agent. In another aspect, the rate of release of the pharmaceutically active agent can be modulated by the presence of an osmagent in the erodible solid.

In additional embodiments, sustained release dosage forms are provided comprising a pharmaceutically active agent and pharmaceutically acceptable salts thereof and adapted to release as an erodible solid over prolonged period of time. The rate of release of the pharmaceutically active agent can be modulated by the presence of an osmagent in the erodible solid.

In yet other embodiments, sustained release dosage forms are provided comprising a pharmaceutically active agent and pharmaceutically acceptable salts thereof, and are adapted to release as an erodible solid over a prolonged period of time. The rate of release of the pharmaceutically active agent in the first hour can be controlled by the amount of osmagent, binding agent and disintegrant present in the erodible solid.

In additional embodiments, sustained release dosage forms are provided comprising a pharmaceutically active agent and pharmaceutically acceptable salts thereof and adapted to release as an erodible solid over a prolonged period of time. The rate of release of the pharmaceutically active agent in the first hour can be controlled by the relative rates of hydration of the osmagent, binding agent and disintegrant present in the erodible solid.

In preferred embodiments, the pharmaceutically active agent is a nonsteroidal anti-inflammatory agent. In particular embodiments, the dosage form provides an immediate release of from 10% to 50% of the nonsteroidal anti-inflammatory agent in the first hour after oral administration of the dosage form, or from 15% to 30% released in the first hour after oral administration. In other particular embodiments, the dosage form provides an immediate release of from 15% to 30% of the nonsteroidal anti-inflammatory agent in the first hour after oral administration of the dosage form. The erodible solid comprises a disintegrant and a binding agent. The erodible solid can also comprise a surfactant and an osmagent. The surfactant can be a nonionic or ionic surfactant. Preferably, the ionic surfactant is an alkali salt of a C₈-C₁₈ alkyl sulfate, and the nonionic surfactant is a poloxamer, or a fatty acid ester of polyoxyethylene, or mixtures thereof.

The dosage form can provide sustained release of active agents for at least 4 hours, more preferably for at least 6-12 hours or longer, and sustained release can be maintained for 12-16 hours if desired. In particular embodiments, the dosage form provides a zero order release from about 1 hour to about 16 hrs after administration, and in certain embodiments, the dosage form provides a zero order release from about 1 hour to about 10 hours after administration. Preferably, the dosage form releases about 90% of the active agent in less than about 12 hrs. In particular embodiments, the dosage form provides a zero order rate of release for at least a portion of the delivery period. In other embodiments, the dosage form provides an ascending rate of release for at least a portion of the delivery period. Preferably, the dosage form provides a faster initial rate of release followed by a zero order rate of release of the remaining active agent, In other preferred embodiments, the dosage form provides a slow initial rate of release followed by an ascending rate of release of the remaining active agent.
In yet other embodiments, the dosage form provides a fast initial rate of release followed by a slower rate of release and an ascending rate of release of the remaining active agent.

Sustained release dosage forms are also disclosed wherein the dosage form comprises: (1) a semipermeable wall defining a cavity and including an exit orifice formed or formable therein; (2) a drug layer comprising a therapeutically effective amount of an active agent such as a nonsteroidal anti-inflammatory agent contained within the cavity and located adjacent to the exit orifice; (3) a push displacement layer contained within the cavity and located distal from the exit orifice; (4) a flow-promoting layer between the inner surface of the semipermeable wall and at least the external surface of the drug layer that is opposite the wall; wherein the dosage form provides an *in vitro* rate of release of the active agent, preferably a nonsteroidal anti-inflammatory agent, for up to about 12 to about 16 hours after being contacted with water in the environment of use. In preferred embodiments, the dosage form further provides an immediate release of the nonsteroidal anti-inflammatory agent without the presence of an immediate release drug coating. In particular embodiment, the dosage form provides an immediate release of from 10% to 50% of the nonsteroidal anti-inflammatory agent in the first hour after oral administration of the dosage form. In additional embodiments, the dosage form provides an immediate release of from 15% to 30% of the nonsteroidal anti-inflammatory agent in the first hour after oral administration of the dosage form.

Methods are also disclosed for providing a sustained release of a pharmaceutically active agent, comprising orally administering to a patient in need thereof at least one embodiment of the sustained release dosage forms described herein. Methods are also disclosed for providing an effective dose of a nonsteroidal anti-inflammatory agent to a patient in need thereof for an extended period of time, comprising orally administering to a patient in need thereof a sustained release dosage form described herein. Methods are also disclosed for controlling the amount and rates or release of a pharmaceutically active agent released from a sustained release dosage form in an immediate release mode and in a sustained release mode.

The sustained release dosage forms can also be used for administering additional pharmaceutically active agents such as opioid analgesics in combination with other active agents. In a preferred embodiment, a nonopioid analgesic is combined with an opioid analgesic in a sustained release dosage form, and release of both agents can be provided at rates proportional to their respective amounts in the dosage form.

The embodiments of the sustained release dosage forms and methods of using them are described in greater detail below.

### Drug coating for immediate release of therapeutic agents

Drug coating formulations can optionally be included in the dosage forms described herein, and provide for the immediate release of active agents along with the sustained release of active agents provided by the sustained release component. Any drug coating formulations known in the art can be used in conjunction with the inventive dosage forms described herein, and can include any pharmaceutical agent, or combinations of agents, whether soluble or insoluble, and at any drug loading. Certain preferred drug coating formulations are described in co-pending commonly owned patent application serial no. 60/506,195, filed as Attorney Docket No. ARC 3363 P1 on September 26, 2003, incorporated by reference herein in its entirety.

For certain preferred drug coatings, briefly, the drug coating can be formed from an aqueous coating formulation and includes at least one insoluble drug and a water soluble film-fomning agent. Two or more insoluble drugs or one or more insoluble drugs in combination with one or more soluble drugs can be included in the drug coating. In a preferred embodiment, the drug coating includes an insoluble drug and a soluble drug. In a preferred embodiment, the insoluble drug included in the drug coating is a nonopioid analgesic, with ibuprofen being a particularly preferred insoluble drug. In an additional preferred embodiment, the soluble drug included in the drug coating is an opioid analgesic, with hydrocodone, oxycodone, hydromorphone, oxymorphone, codeine and methadone being particularly preferred soluble drugs.

In preferred embodiments, the drug coating includes from 85 wt% to 97 wt% insoluble drug, with coatings exhibiting an insoluble drug loading of 90 wt% to 93 wt% being particularly preferred. The total amount of soluble drug included in the drug coating preferably ranges from 0.5 wt% to 15 wt% soluble drug, and drug coaxings including 1 wt% to 3 wt% soluble drug being most preferred. The total amount of insoluble drug included in a drug coating that incorporates both soluble and insoluble drugs preferably ranges from 60 wt% to 96.5 wt%, with drug coatings including 75 wt% to 89.5 wt% insoluble drug being more preferred, and drug coatings including 89 wt% to 90 wt% insoluble drug being most preferred. The total amount of drugs included in the drug coating ranges from 85 wt% to 97 wt%, and in preferred embodiments, the total amount of drug included in a drug coating ranges from 90 wt% to 93 wt %.

In one preferred embodiment, the insoluble drug included in the drug coating is a nonopioid analgesic. Preferred nonopioid analgesics include ibuprofen and acetaminophen, among others. In an additional preferred embodiment, the soluble drug included in the drug coating is an opioid analgesic, with hydrocodone, oxycodone, hydromorphone, oxymorphone, codeine and methadone being particularly preferred soluble drugs.

The film-forming agent included in the drug coating is water soluble and accounts for 3 wt% to 15 wt% of the drug coating, with drug coatings having 7 wt% to 10 wt% film-forming agent being preferred. The film-forming agent included in a drug coating is water soluble and preferably works to solubilize insoluble drug included in the drug coating. In addition, the film-forming agent included in a drug coating may be chosen such that the film-forming agent forms a solid solution with one or more insoluble drugs included in the drug coating. It is believed that drug loading and film forming characteristics of a drug coating are enhanced by selecting a film-forming agent that forms a solid solution with at least one of the one or more insoluble drugs included in the drug coating. A drug dissolved at the molecular level within the film-forming agent (a solid solution) is also expected to be more readily bioavailable because, as the drug coating breaks down or dissolves, the drug is released into the gastrointestinal tract and presented to the gastrointestinal mucosal tissue as discrete molecules.

In a preferred embodiment, the film-forming agent included in the drug coating is a film-forming polymer or a polymer blend including at least one film-forming polymer. Polymer materials used as the film-forming agent of a drug coating are water soluble. Examples of water soluble polymer materials that may be used as the film-forming polymer of a drug coating include, but are not limited to, hydroxypropylmethyl cellulose ("HPMC"), low molecular weight HPMC, hydroxypropyl cellulose ("HPC") (*e*.*g*., Klucel^{®}), hydroxyethyl cellulose ("HEC") (*e*.*g*., Natrasol^{®}), copovidone (*e*.*g*., Kollidon^{®} VA 64), and PVA-PEG graft copolymer (*e.g*., Kollicoat^{®} IR), and combinations thereof. A polymer blend or mixture may be used as the film forming agent in order to achieve a drug coating having characteristics that may not be achievable using a single film-forming polymer in combination with the drug or drugs to be included in the drug coating. For example, blends of HPMC and copovidone provide a film-forming agent that allows the formation of drug coatings that not only exhibit desirable drug loading characteristics, but also provide coatings that are aesthetically pleasing and exhibit desirable physical properties.

The drug coating can also include a viscosity enhancer. Because the drug coating is an aqueous coating that includes an insoluble drug, the drug coating is typically coated from an aqueous suspension formulation. In order to provide a drug coating with substantially uniform drug distribution from a suspension formulation, however, the suspension formulation should provide a substantially uniform dispersion of the insoluble drug included in the coating. Depending on the relative amounts and nature of the film-forming agent and the drugs included in a drug coating, a viscosity enhancer can be included in a drug coating to facilitate the creation of a coating formulation that exhibits sufficient viscosity to provide a substantially uniform drug dispersion and facilitates the production of a drug coating having a substantially uniform distribution of insoluble drug. A viscosity enhancer included in a drug coating is preferably water-soluble and can be a film-forming agent. Examples of viscosity enhancers that may be used in a drug coating include, but are not limited to, HPC (*e*.*g*., Klucel^{®}), HEC (*e*.*g*., Natrasol^{®}), Polyox^{®} water soluble resin products, and combinations thereof.

The precise amount of viscosity enhancing material included in the drug coating will vary, depending on the amounts and type of film-forming polymer and drug materials to be used in the drug coating. However, where included in a drug coating, a viscosity enhancer will typically account for 5 wt%, or less, of the drug coating. Preferably, a drug coating includes 2 wt%, or less, viscosity enhancer, and in particularly preferred embodiments, the drug coating includes 1 wt%, or less, viscosity enhancer.

The drug coating can also include a disintegrating agent that increases the rate at which the drug coating disintegrates after administration. Because the drug coating typically includes a large amount of insoluble drug, the drug coating may not break down or disintegrate as rapidly as desired after administration. A disintegrating agent included in a coating is a water swellable material that works to structurally compromise the coating as the disintegrating agent absorbs water and swells. Disintegrating agents that may be used in the drug coating include, but are not limited to modified starches, modified cellulose, and cross-linked polyvinylpyrrolidone materials. Specific examples of disintegrating agents that may be used in the drug coating and are commercially available include Ac-Di-Sol^{®}, Avicel^{®}, and PVP XL-10. Where included in the drug coating, a disintegrating agent typically accounts for up to about 6 wt% of the coating, with coatings incorporating from 0.5 wt% to 3 wt% being preferred and coatings incorporating from 1 wt% to 3 wt% being particularly preferred.

The drug coating can also include a surfactant to increase the rate at which the drug coating dissolves or erodes after administration. The surfactant serves as a "wetting" agent that allows aqueous liquids to more easily spread across or penetrate the drug coating. Surfactants suitable for use in a drug coating are preferably solid at 25°C. Examples of surfactants that may be used in the drug coating include, but are not limited to, surface active polymers, such as Poloxamer and Pluronic^{®} surfactants. Where a surfactant is included in a drug coating, the surfactant will typically account for up to about 6 wt% of the drug coating, with drug coatings including about 0.5 wt% to about 3 wt% surfactant being preferred, and drug coatings including about 1 wt% to about 3 wt% surfactant being particularly preferred.

In one embodiment of the drug coating, the film-forming agent includes a polymer blend formed of copovidone and HPMC. Where such a polymer blend is used as the film-forming agent of the drug coating, the amounts of copovidone and HPMC can vary, as desired, to achieve a drug coating having desired physical and drug-loading characteristics. However, where the film-agent included in a drug coating is formed of a blend of copovidone and HPMC, the copovidone and HPMC are preferably included at a wt/wt ratio 0.6:1 to 0.7:1 copovidone to HPMC, with a wt/wt ratio of 1:1.5 being most preferred. Blends of HPMC and copovidone provide drug coatings that are aesthetically pleasing and are believed to be sufficiently robust to withstand further processing and an extended shelf life. Moreover, it is believed that copovidone can work to solubilize insoluble drug included in a drug coating, providing a drug coating that includes a solid solution of insoluble drug.

In another embodiment, the drug coating includes a blend of HPMC and copovidone as the film-forming agent, an insoluble drug, and a soluble drug. In a specific example of such an embodiment, the drug coating can include an insoluble drug such as a nonopioid analgesic and a soluble drug such as an opioid analgesic. A dosage form that includes the combination of a nonopioid analgesic and an opioid analgesic provides a combination of analgesic, anti-inflammatory, anti-pyretic, and antitussive actions.

In even further embodiments, the drug coating includes a blend of HPMC and copovidone as the film-forming agent, an insoluble nonopioid analgesic, a soluble opioid analgesic, and a viscosity enhancing agent or a disintegrating agent. In a specific example of such an embodiment, the drug coating includes between 1 wt% and 2 wt% of a viscosity enhancing agent, such as HPC. In another example of such an embodiment, the drug coating includes between 0.5 wt% and 3 wt% disintegrating agent, and in yet another example of such an embodiment, the drug coating includes between 0.5 wt% and 3 wt% of a surfactant.

The drug coating is not only capable of achieving high drug loading, but where the drug coating includes two or more different drugs, it has been found that the drug coating releases the different drugs in amounts that are directly proportional to the amounts of the drugs included in the drug coating. The proportional release is observed even where drugs exhibiting drastically different solubility characteristics, such as acetaminophen and hydrocodone, are included in the drug coating. In addition a drug coating releases substantially all of the drug included therein. Such performance characteristics facilitate reliable and predictable drug delivery performance, and allow formulation of drug coatings that deliver two or more drugs at a wide range of different ratios.

In another aspect, a coating formulation can be used to provide a drug coating. The coating suspension includes the materials used to form a drug coating which is dissolved or suspended, depending on the material, within one or more solvents or solutions. The one or more solvents included in a coating suspension are not organic solvents, and are preferably aqueous solvents. Aqueous solvents that may be used in a coating suspension include, but are not limited to, purified water, pH adjusted water, acidified water, or aqueous buffer solutions. In a preferred embodiment, the aqueous solvent included in a coating suspension is purified water USP. The coating formulation is preferably an aqueous formulation and avoids the potential problems and disadvantages that can result from the use of organic solvents in formulating coating compositions.

As the drug coating includes at least one insoluble drug, the coating formulation is typically prepared as an aqueous suspension using any suitable process, and in preferred embodiments the coating formulation is formulated to facilitate production of drug coatings through a known coating process, such as, for example, pan coating, fluid bed coating, or any other standard coating processes suitable for providing a drug coating. Though the precise amount of solvent used in a coating suspension may vary depending on, for example, the materials to be included in the finished drug coating, the desired coating performance of the coating suspension and the desired physical characteristics of the finished drug coating, a coating suspension typically includes up to about 30 wt% solids content, with the remainder of the coating suspension consisting of the desired solvent. A preferred embodiment of a coating suspension includes about 80 wt% of a desired aqueous solvent and about 20 wt% solids content. The coating suspension is formulated to exhibit a viscosity that is low enough to facilitate spray coating of drug coating, yet is high enough to maintain a substantially uniform dispersion of the insoluble drug included in the coating suspension during a coating process.

In preparing a coating formulation, the drug loaded into the coating formulation can be provided in micronized form. By reducing the particle size of the drug loaded into a coating formulation, a more cosmetically smooth drug coating may be achieved. In addition, by reducing the particle size of the drug material loaded into a coating formulation, the dissolution rate of the drug when released from the drug coating prepared by the coating formulation may be improved, particularly where the drug is an insoluble drug. In one embodiment of the coating formulation, the coating formulation includes a micronized drug material exhibiting an average particle size of less than 100 microns. In another embodiment, the coating formulation includes a micronized drug material exhibiting an average particle size of less than 50 microns, and in yet another embodiment, the coating formulation includes a micronized drug material exhibiting an average particle size of less than 10 microns. Micronization of the drug material can be readily achieved through processes well known in the art, such as, for example, known bead milling, jet milling or microprecipitation processes, and particle size can be measured using any conventional particle size measuring technique, such as sedimentation field flow fractionation, photon correlation spectroscopy or disk centrifugation.

The solids dissolved or suspended in a coating formulation are loaded into the coating formulation in the same relative amounts as are used in a drug coating. For example, the drug included in a coating formulation accounts for 85 wt% to 97 wt% of the solids loaded into the coating formulation. In preferred embodiments, the drug included in a coating formulation accounts for 90 wt% to 93 wt% of the solids loaded into the coating formulation. The film-forming agent included in a coating formulation accounts for 3 wt% to 15 wt% of the solids loaded into the coating formulation, and in preferred embodiments, the film forming agent included in a coating formulation accounts for 7 wt% to 10 wt% of the solids loaded into the coating formulation. Where included, a viscosity enhancer will typically account for 5 wt%, or less, of the solids included in a coating formulation. Coating formulations wherein the viscosity enhancer accounts for 2 wt%, or less, of the solids are preferred, and in particularly preferred embodiments, a viscosity enhancer included in a coating formulation accounts for 1 wt%, or less, of the solids included in the coating formulation. If the coating to be formed by the coating formulation is to include a disintegrating agent, the disintegrating agent typically accounts for up to about 6 wt% of the solids included in the coating formulation. In preferred embodiments, a disintegrating agent will account for. 0.5 wt% to 3 wt% of the solids included in the coating formulation, and in particularly preferred embodiments of a coating formulation including a disintegrating agent, the disintegrating agent accounts for 1 wt% to 3 wet% of the solids included in the coating formulation. Where a surfactant is included in a drug coating according to the present invention, the surfactant will typically account for up to about 6 wt% of the solids included in the coating formulation. Preferably, if a surfactant is included in a coating formulation, the surfactant will account for 0.5 wt% to 3 wt% of the solids included in the coating formulation, and in particularly preferred embodiments of a coating formulation that includes a surfactant, the surfactant accounts for 1 wt% to 3 wt% of the solids included in the coating formulation.

### Sustained release dosage forms containing pharmaceutically active agents

The OROS^{®} technology provides tunable sustained release dosage forms that can provide sustained release of one or more active agents, with or without the use of a drug coating providing immediate release of drug. Various types of osmotic dispensers include elementary osmotic pumps, such as those described in U.S. Patent No. 3,845,770, mini-osmotic pumps such as those described in U.S. Patent Nos. 3,995,631, 4,034,756 and 4,111,202, and multi-chamber osmotic systems referred to as push-pull, push-melt and push-stick osmotic pumps, such as those described in U.S. Patent Nos. 4,320,759, 4,327,725, 4,449,983, 4,765, 989 and 4,940,465, 6,368,626 to Bhatt. Specific adaptations of OROS^{®} that can be used preferably include the OROS^{®} Push-Stick^{™} System. A significant advantage to osmotic systems is that operation is substantially pH-independent and thus continues at the osmotically determined rate throughout an extended time period even as the dosage form transits the gastrointestinal tract and encounters differing microenvironments having significantly different pH values. Sustained release can be provided for times as short as a few hours or for as long as the dosage form resides in the gastrointestinal tract.

Osmotic dosage forms utilize osmotic pressure to generate a driving force for imbibing fluid into a compartment formed, at least in part, by a semi-permeable wall that permits diffusion of water but not drug or osmagents, if present. In these Osmotic dosage forms, the active agent reservoir(s) is typically formed with an active agent compartment, containing a pharmaceutical agent in the form of a solid, liquid or suspension, as the case may be, and an expandable "push" compartment of a hydrophilic polymer that will imbibe fluid from the stomach, swell and force the active agent out of the dosage form and into the environment of use.

A review of such osmotic dosage forms is found in Santus and Baker (1995), "Osmotic drug delivery: a review of the patent literature," Journal of Controlled Release 35: 1-21. In particular, the following U.S. Patents, owned by the assignee of the present application, ALZA Corporation, and directed to osmotic dosage forms, are each incorporated in their entirety herein: U.S. Patent Nos. 3,845,770; 3,916,899; 3,995,631; 4,008,719; 4,111,202; 4,160,020; 4,327,725; 4,519,801; 4,578,075; 4,681,583; 5,019,397; 5,156,850; 5,912,268; 6,375,978; 6,368,626; 6,342,249; 6,333,050; 6,287,295; 6,283,953; 6,270,787; 6,245,357; and 6,132,420.

The core of the dosage form typically comprises a drug layer comprising a dry composition or substantially dry composition formed by compression of the binding agent and the analgesic agents as one layer and the expandable or push layer as the second layer. By "dry composition" or "substantially dry composition" is meant that the composition forming the drug layer of the dosage form is expelled from the dosage form in a plug-like state, the composition being sufficiently dry or so highly viscous that it does not readily flow as a liquid stream from the dosage form under the pressure exerted by the push layer. The drug layer itself has very little osmotic activity relative to the push layer, as the drug, binding agent and disintegrant are not well hydrated, and the drug layer does not flow out of the dosage form as a slurry or suspension. The drug layer is exposed to the environment of use as an erodible composition, in contrast to alternative osmotic dosage forms in which the drug layer is exposed to the environment of use as a slurry or suspension. The drug layer is an erodible composition because it includes very little if any osmagent due to the high drug loading provided as well as the poor solubility of the drug to be delivered.

The core of the dosage form typically comprises a drug layer comprising a dry composition formed by compression of the binding agent and the analgesic agents as one layer and the expandable or push layer as the second layer. By "dry composition" or "substantially dry composition" is meant that the composition forming the drug layer of the dosage form is expelled from the dosage form in a plug-like state, the composition being sufficiently dry or so highly viscous that it does not readily flow as a liquid stream from the dosage form under the pressure exerted by the push layer. The drug layer itself has very little osmotic activity relative to the push layer, as the drug, binding agent and disintegrant are not well hydrated, and the drug layer does not flow out of the dosage form as a slurry or suspension. The drug layer is exposed to the environment of use as an erodible composition, in contrast to alternative osmotic dosage forms in which the drug layer is exposed to the environment of use as a slurry or suspension. The drug layer is an erodible composition because it includes very little if any osmagent due to the high drug loading provided as well as the poor solubility of the drug to be delivered.

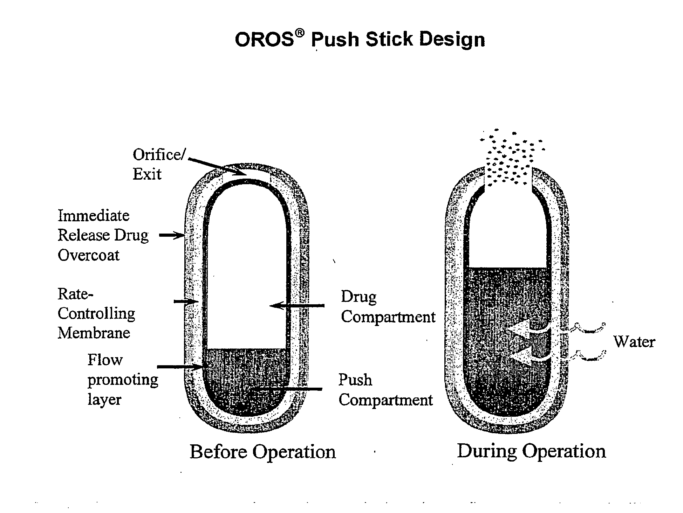

Compression techniques are known in the art and exemplified in Example 1. The expandable layer pushes the drug layer from the exit orifice as the push layer imbibes fluid from the environment of use, and the exposed drug layer will be eroded to release the drug into the environment of use. This may be seen with reference to FIG. 1. Upon release from the dosage form, the drug layer imbibes water causing the disintegrant to swell and soluble agents to dissolve, allowing the erodible solid to disperse and the analgesic agents to dissolve in the fluid at the environment of use. This "push-stick" formulation is a preferred dosage form and is described in greater detail below.

A particular embodiment of the osmotic dosage form comprises: a semipermeable wall defining a cavity and including an exit orifice formed or formable therein, a drug layer comprising a therapeutically effective amount of at least one pharmaceutically active agent (e.g., an opioid analgesic and a nonopioid analgesic) contained within the cavity and located adjacent to the exit orifice, a push displacement layer contained within the cavity and located distal from the exit orifice, and a flow-promoting layer between the inner surface of the semipermeable wall and at least the external surface of the drug layer that is opposite the wall. The dosage form provides an *in vitro* rate of release of the opioid analgesic and the nonopioid analgesic for up to about 12 hours or longer after being contacted with water in the environment of use.

### Composition of the osmotic dosage forms

A preferred embodiment of a dosage form of this invention having the "push-stick" configuration is illustrated in FIG. 1 prior to its administration to a subject, during operation and after delivery of the active agent. The dosage form comprises a wall defining a cavity and an exit orifice. Within the cavity and remote from the exit orifice is a push displacement layer, and a drug layer is located within cavity adjacent the exit orifice. A flow-promoting layer extends at least between the drug layer and the inner surface of the wall, and can extend between the inner surface of the wall and the push displacement layer.

The dosage form is typically at high drug loading, i.e., 60% or greater, but more generally 70% or greater, active agent in the drug layer based on the overall weight of the drug layer, and is exposed to the environment of use as an erodible composition. The drug layer comprises a composition formed of a relatively insoluble drug and can be combined with additional drugs with the same or differing solubility. A particular embodiment includes an opioid analgesic and nonopioid analgesic in combination with a disintegrant, a binding agent, optionally a surfactant and/or osmagent, and mixtures thereof. The binding agent is generally a hydrophilic polymer that contributes to the release rate of active agent and controlled delivery pattern, such as a hydroxyalkylcellulose, a hydroxypropylalkylcellulose, a poly(alkylene) oxide, or a polyvinylpyrrolidone, or mixtures thereof. These hydrophilic polymers become hydrated in the presence of water at varying rates, depending on their chemical substitution and molecular weights. Representative examples of these hydrophilic polymers are poly(alkylene oxides) of 100,000 to 750,000 number-average molecular weight, including without limitation poly(ethylene oxide), poly(methylene oxide), poly(butylene oxide) and poly(hexylene oxide); poly(carboxymethylcelluloses) of 40,000 to 400,000 number-average molecular weight, represented by poly(alkali carboxymethylcellulose), such as poly(sodium carboxymethylcellulose), poly(potassium carboxymethylcellulose) and poly(lithium carboxymethylcellulose); hydroxyalkylcelluloses of 9,200 to 125,000 number-average molecular weight such as hydroxypropylcellulose, hydroxypropylalkylcelluloses such as hydroxypropylalkylcellulose of 9,200 to 125,000 number-average molecular weight, including without limitation, hydroxypropylethylcellulose, hydroxypropyl methylcellulose, hydroxypropylbutylcellulose and hydroxypropylpentylcellulose; and poly(vinylpyrrolidones) of 7,000 to 75,000 number-average molecular weight. Preferred among those polymers are the poly(ethylene oxide) of 100,000-300,000 number average molecular weight, poly(vinylpyrrolidones) of 7,000 to 75,000 number-average molecular weight and hydroxyalkylcelluloses. For example, poly(vinylpyrrolidones) are known as fast hydrating polymers, while hydroxyalkylcelluloses, particularly hydroxypropylcellulose, are slow hydrating polymers. Carriers that erode in the gastric environment, i.e., bioerodible carriers, are especially preferred.

Surfactants and disintegrants may be utilized in the carrier as well. Disintegrants generally include starches, clays, celluloses, algins and gums and crosslinked starches, celluloses and polymers. Representative disintegrants include corn starch, potato starch, croscarmellose sodium, crospovidone, sodium starch glycolate, Veegum HV, methylcellulose, agar, bentonite, carboxymethylcellulose, low substituted carboxymethylcellulose, alginic acid, guar gum and the like. A preferred disintegrant is croscarmellose sodium.

Exemplary surfactants are those having an HLB value of between about 10-25, such as polyethylene glycol 400 monostearate, polyoxyethylene-4-sorbitan monolaurate, polyoxyethylene-20-sorbitan monooleate, polyoxyethylene-20-sorbitan monopalmitate, polyoxyethylene-20-monolaurate, polyoxyethylene-40-stearate, sodium oleate and the like. Surfactants that are useful generally include ionic surfactants, including anionic, cationic, and zwitterionic surfactants, and nonionic surfactants. Nonionic surfactants are preferred in certain embodiments and include, for example, polyoxyl stearates such as polyoxyl 40 stearate, polyoxyl 50 stearate, polyoxyl 100 stearate, polyoxyl 12 distearate, polyoxyl 32 distearate, and polyoxyl 150 distearate, and other Myrj™ series of surfactants, or mixtures thereof. Yet another class of surfactant useful in forming the dissolved drug are the triblock co-polymers of ethylene oxide/propylene oxide/ethylene oxide, also known as poloxamers, having the general formula HO(C₂H₄O)ₐ(-C₃H₆O)_{b}(C₂H₄O)ₐH, available under the tradenames Pluronic and Poloxamer. In this class of surfactants, the hydrophilic ethylene oxide ends of the surfactant molecule and the hydrophobic midblock of propylene oxide of the surfactant molecule serve to dissolve and suspend the drug. These surfactants are solid at room temperature. Other useful surfactants include sugar ester surfactants, sorbitan fatty acid esters such as sorbitan monolaurate, sorbitan monopalmitate, sorbitan monostearate, sorbitan tristearate, and other Span^{™} series surfactants, glycerol fatty acid esters such as glycerol monostearate, polyoxyethylene derivatives such as polyoxyethylene ethers of high molecular weight aliphatic alcohols (e.g., Brij 30, 35, 58, 78 and 99) polyoxyethylene stearate (self emulsifying), polyoxyethylene 40 sorbitol lanolin derivative, polyoxyethylene 75 sorbitol lanolin derivative, polyoxyethylene 6 sorbitol beeswax derivative, polyoxyethylene 20 sorbitol beeswax derivative, polyoxyethylene 20 sorbitol lanolin derivative, polyoxyethylene 50 sorbitol lanolin derivative, polyoxyethylene 23 lauryl ether, polyoxyethylene 2 cetyl ether with butylated hydroxyanisole, polyoxyethylene 10 cetyl ether, polyoxyethylene 20 cetyl ether, polyoxyethylene 2 stearyl ether, polyoxyethylene 10 stearyl ether, polyoxyethylene 20 stearyl ether, polyoxyethylene 21 stearyl ether, polyoxyethylene 20 oleyl ether, polyoxyethylene 40 stearate, polyoxyethylene 50 stearate, polyoxyethylene 100 stearate, polyoxyethylene derivatives of fatty acid esters of sorbitan such as polyoxyethylene 4 sorbitan monostearate, polyoxyethylene 20 sorbitan tristearate, and other Tween^{™} series of surfactants, phospholipids and phospholipid fatty acid derivatives such as lecithins, fatty amine oxides, fatty acid alkanolamides, propylene glycol monoesters and monoglycerides, such as hydrogenated palm oil monoglyceride, hydrogenated soybean oil monoglyceride, hydrogenated palm stearine monoglyceride, hydrogenated vegetable monoglyceride, hydrogenated cottonseed oil monoglyceride, refined palm oil monoglyceride, partially hydrogenated soybean oil monoglyceride, cotton seed oil monoglyceride sunflower oil monoglyceride, sunflower oil monoglyceride, canola oil monoglyceride, succinylated monoglycerides, acetylated monoglyceride, acetylated hydrogenated vegetable oil monoglyceride, acetylated hydrogenated coconut oil monoglyceride, acetylated hydrogenated soybean oil monoglyceride, glycerol monostearate, monoglycerides with hydrogenated soybean oil, monoglycerides with hydrogenated palm oil, succinylated monoglycerides and monoglycerides, monoglycerides and rapeseed oil, monoglycerides and cottonseed oils, monoglycerides with propylene glycol monoester sodium stearoyl lactylate silicon dioxide, diglycerides, triglycerides, polyoxyethylene steroidal esters, Triton-X series of surfactants produced from octylphenol polymerized with ethylene oxide, where the number "100" in the trade name is indirectly related to the number of ethylene oxide units in the structure, (e.g., Triton X-100^{™} has an average ofN = 9.5 ethylene oxide units per molecule, with an average molecular weight of 625) and having lower and higher mole adducts present in lesser amounts in commercial products, as well as compounds having a similar structure to Triton X-100^{™}, including Igepal CA-630^{™} and Nonidet P-40M (NP-40^{™}, N-lauroylsarcosine, Sigma Chemical Co., St. Louis, Mo.), and the like. Any of the above surfactants can also include optional added preservatives such as butylated hydroxyanisole and citric acid. In addition, any hydrocarbon chains in the surfactant molecules can be saturated or unsaturated, hydrogenated or unhydrogenated.

An especially preferred family of surfactants are the poloxamer surfactants, which are a:b:a triblock co-polymers of ethylene oxide:propylene oxide:ethylene oxide. The "a" and "b" represent the average number of monomer units for each block of the polymer chain. These surfactants are commercially available from BASF Corporation of Mount Olive, New Jersey, in a variety of different molecular weights and with different values of "a" and "b" blocks. For example, Lutrol^{®} F127 has a molecular weight range of 9,840 to 14,600 and where "a" is approximately 101 and "b" is approximately 56, Lutrol F87 represents a molecular weight of 6,840 to 8,830 where "a" is 64 and "b" is 37, Lutrol F108 represents an average molecular weight of 12,700 to 17,400 where "a" is 141 and "b" is 44, and Lutrol F68 represents an average molecular weight of 7,680 to 9,510 where "a" has a value of about 80 and "b" has a value of about 27.

Other surfactants are the sugar ester surfactants, which are sugar esters of fatty acids. Such sugar ester surfactants include sugar fatty acid monoesters, sugar fatty acid diesters, triesters, tetraesters, or mixtures thereof, although mono- and di-esters are most preferred. Preferably, the sugar fatty acid monoester comprises a fatty acid having from 6 to 24 carbon atoms, which may be linear or branched, or saturated or unsaturated C₆ to C₂₄ fatty acids. The C₆ to C₂₄ fatty acids include C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, and C₂₄ in any subrange or combination. These esters are preferably chosen from stearates, behenates, cocoates, arachidonates, palmitates, myristates, laurates, carprates, oleates, laurates and their mixtures.

Preferably, the sugar fatty acid monoester comprises at least one saccharide unit, such as sucrose, maltose, glucose, fructose, mannose, galactose, arabinose, xylose, lactose, sorbitol, trehalose or methylglucose. Disaccharide esters such as sucrose esters are most preferable, and include sucrose cocoate, sucrose monooctanoate, sucrose monodecanoate, sucrose mono- or dilaurate, sucrose monomyristate, sucrose mono- or dipalmitate, sucrose mono- and distearate, sucrose mono-, di- or trioleate, sucrose mono- or dilinoleate, sucrose polyesters, such as sucrose pentaoleate, hexaoleate, heptaoleate or octooleate, and mixed esters, such as sucrose palmitate/stearate.

Particularly preferred examples of these sugar ester surfactants include those sold by the company Croda Inc of Parsippany, NJ under the names Crodesta F10, F50, F160, and F110 denoting various mono-, di- and mono/di ester mixtures comprising sucrose stearates, manufactured using a method that controls the degree of esterification, such as described in U.S. Patent No. 3,480,616. These preferred sugar ester surfactants provide the added benefit of tableting ease and nonsmearing granulation.

Use may also be made of those sold by the company Mitsubishi under the name Ryoto Sugar esters, for example under the reference B370 corresponding to sucrose behenate formed of 20% monoester and 80% di-, tri- and polyester. Use may also be made of the sucrose mono- and dipalmitate/stearate sold by the company Goldschmidt under the name "Tegosoft PSE". Use may also be made of a mixture of these various products. The sugar ester can also be present in admixture with another compound not derived from sugar; and a preferred example includes the mixture of sorbitan stearate and of sucrose cocoate sold under the name "Arlatone 2121" by the company ICI. Other sugar esters include, for example, glucose trioleate, galactose di-, tri-, tetra- or pentaoleate, arabinose di-, tri- or tetralinoleate or xylose di-, tri- or tetralinoleate, or mixtures thereof. Other sugar esters of fatty acids include esters of methylglucose include the distearate of methylglucose and of polyglycerol-3 sold by the company Goldschmidt under the name of Tegocare 450. Glucose or maltose monoesters can also be included, such as methyl O-hexadecanoyl-6-D-glucoside and O-hexadecanoyl-6-D-maltose. Certain other sugar ester surfactants include oxyethylenated esters of fatty acid and of sugar include oxyethylenated derivatives such as PEG-20 methylglucose sesquistearate, sold under the name "Glucamate SSE20", by the company Amerchol.

A resource of surfactants including solid surfactants and their properties is available in McCutcheon's Detergents and Emulsifiers, International Edition 1979 and McCutcheon's Detergents and Emulsifiers, North American Edition 1979. Other sources of information on properties of solid surfactants include BASF Technical Bulletin Pluronic & Tetronic Surfactants 1999 and General Characteristics of Surfactants from ICI Americas Bulletin 0-1 10/80 5M, and Eastman Food Emulsifiers Bulletin ZM-1K October 1993.

One of the characteristics of surfactants tabulated in these references is the HLB value, or hydrophilic lipophilic balance value. This value represents the relative hydroplicility and relative hydrophobicity of a surfactant molecule. Generally, the higher the HLB value, the greater the hydrophilicity of the surfactant while the lower the HLB value, the greater the hydrophobicity. For the Lutrol^{®} molecules, for example, the ethylene oxide fraction represents the hydrophilic moiety and the propylene oxide fraction represents the hydrophobic fraction. The HLB values of Lutrol F127, F87, F108, and F68 are respectively 22.0, 24.0, 27.0, and 29.0. The preferred sugar ester surfactants provide HLB values in the range of about 3 to about 15. The most preferred sugar ester surfactant, Crodesta F160 is characterized by having a HLB value of 14.5.

Ionic surfactants include cholic acids and derivatives of cholic acid such as deoxycholic acid, ursodeoxycholic acid, taurocholic acid, taurodeoxycholic acid, taurochenodeoxycholic acid, and salts thereof, and anionic surfactants, the most common example of which is sodium dodecyl (or lauryl) sulfate. Zwitterionic or amphoteric surfactants generally include a carboxylate or phosphate group as the anion and an amino or quaternary ammonium moiety as the cation. These include, for example, various polypeptides, proteins, alkyl betaines, and natural phospholipids such as lecithins and cephalins, alkyl-beta-aminopropionates and 2-alkyl-imidazoline quaternary ammonium salts, as well as the CHAPS series of surfactants (e.g., 3-[3-Cholamidopropyl) dimethylammoniol]-1-propanesulfonate hydrate available from Aldrich), and the like.

Surfactants typically have poor cohesive properties and therefore do not compress as hard, durable tablets. Furthermore, surfactants are in the physical form of liquid, pastes, or waxy solids at standard temperatures and conditions and are inappropriate for tableted oral pharmaceutical dosage forms. The aforementioned surfactants have been surprisingly found to function by enhancing the solubility and potential bioavailability of low solubility drugs delivered in high doses.

Surfactant can be included as one surfactant or as a blend of surfactants. The surfactants are selected such that they have values that promote the dissolution and solubility of the drug. A high HLB surfactant can be blended with a surfactant of low HLB to achieve a net HLB value that is between them, if a particular drug requires the intermediate HLB value. The surfactant is selected depending upon the drug being delivered; such that the appropriate HLB grade is utilized.

The relatively insoluble drug (.e.g., a nonopioid analgesic) can be provided in the drug layer in amounts of from about 1 microgram to about 1000 mg per dosage form, and more typically from about 200 to about 600 mg, depending upon the required dosing level that must be maintained over the delivery period, i.e., the time between consecutive administrations of the dosage forms. In a preferred embodiment, the nonopioid analgesic is ibuprofen at 200 mg to 600 ± 100 mg. Generally, loading of compound in the dosage forms will provide doses of the nonopioid analgesic to a subject ranging up to about 3000 mg per day, more usually up to about 1000 to 2000 mg per day, depending on the level of pain being experienced by the patient.

The additional active agent (e.g., an opioid analgesic) can be provided in the drug layer in amounts of from 1 microgram to 500 mg per dosage form, and more typically from about 10 to about 100 mg, depending upon the required dosing level that must be maintained over the delivery period, i.e., the time between consecutive administrations of the dosage forms. In a preferred embodiment, the opioid analgesic is hydrocodone at 15 ± 5 mg. Generally, loading of compound in the dosage forms will provide doses of the active agent to a subject ranging up to about 2000 mg per day, more between about 10 to 60 or 600 mg per day, depending on the level of medication required by the patient.

The push layer is an expandable layer having a push-displacement composition in direct or indirect contacting layered arrangement with the drug layer. The push layer generally comprises a polymer that imbibes an aqueous or biological fluid and swells to push the drug composition through the exit means of the device. Representatives of fluid-imbibing displacement polymers comprise members selected from poly(alkylene oxide) of 1 million to 15 million number-average molecular weight, as represented by poly(ethylene oxide) and poly(alkali carboxymethylcellulose) of 500,000 to 3,500,000 number-average molecular weight, wherein the alkali is sodium, potassium or lithium. Examples of additional polymers for the formulation of the push-displacement composition comprise osmopolymers comprising polymers that form hydrogels, such as Carbopol^{®} acidic carboxypolymer, a polymer of acrylic cross-linked with a polyallyl sucrose, also known as carboxypolymethylene, and carboxyvinyl polymer having a molecular weight of 250,000 to 4,000;000; Cyanamer^{®} polyacrylamides; cross-linked water swellable indenemaleic anhydride polymers; Good-rite^{®} polyacrylic acid having a molecular weight of 80,000 to 200,000; Aqua-Keeps^{®} acrylate polymer polysaccharides composed of condensed glucose units, such as diester cross-linked polygluran; and the like. Representative polymers that form hydrogels are known to the prior art in U.S. Patent No. 3,865,108, issued to Hartop; U.S. Patent No. 4,002,173, issued to Manning; U.S. Patent No. 4,207,893, issued to Michaels; and in Handbook of Common Polymers, Scott and Roff, Chemical Rubber Co., Cleveland, Ohio.

The osmagent, also known as osmotic solute and osmotically effective agent, which exhibits an osmotic pressure gradient across the outer wall and subcoat, comprises a member selected from the group consisting of sodium chloride, potassium chloride, lithium chloride, magnesium sulfate, magnesium chloride, potassium sulfate, sodium sulfate, lithium sulfate, potassium acid phosphate, mannitol, urea, inositol, magnesium succinate, tartaric acid raffinose, sucrose, glucose, lactose, sorbitol, inorganic salts, organic salts and carbohydrates. Low molecular weight sugars such as mannitol and sorbitol are described as osmagents in the examples.

A flow promoting layer (also called the subcoat for brevity) is in contacting relationship with the inner surface of the semipermeable wall and at least the external surface of the drug layer that is opposite wall; although the flow-promoting layer may, and preferably will, extend to, surround and contact the external surface of the push displacement layer. The wall typically will surround at least that portion of the external surface of the drug layer that is opposite the internal surface of the wall. The flow-promoting layer may be formed as a coating applied over the compressed core comprising the drug layer and the push layer. The outer semipermeable wall surrounds and encases the inner flow-promoting layer. The flow-promoting layer is preferably formed as a subcoat of at least the surface of the drug layer, and optionally the entire external surface of the compacted drug layer and the push displacement layer. When the semipermeable wall is formed as a coat of the composite formed from the drug layer, the push layer and the flow-promoting layer, contact of the semipermeable wall with the flow-promoting layer is assured.

The flow-promoting layer facilitates release of drug from the dosage forms of the invention by reducing the frictional forces between the semipermeable wall 2 and the outer surface of the drug layer, thus allowing for more complete delivery of drug from the device. Particularly in the case of active compounds having a high cost, such an improvement presents substantial economic advantages since it is not necessary to load the drug layer with an excess of drug to insure that the minimal amount of drug required will be delivered.

The flow-promoting layer typically may be 0.01 to 5 mm thick, more typically 0.5 to 5 mm thick, and it comprises a member selected from hydrogels, gelatin, low molecular weight polyethylene oxides (e.g., less than 100,000 MW), hydroxyalkylcelluloses (e.g., hydroxyethylcellulose), hydroxypropylcelluloses, hydroxyisopropylcelluoses, hydroxybutylcelluloses and hydroxyphenylcelluloses, and hydroxyalkyl alkylcelluloses (e.g., hydroxypropyl methylcellulose), and mixtures thereof. The hydroxyalkylcelluloses comprise polymers having a 9,500 to 1,250,000 number-average molecular weight. For example, hydroxypropyl celluloses having number average molecular weights of between 80,000 to 850,000 are useful. The flow promoting layer may be prepared from conventional solutions or suspensions of the aforementioned materials in aqueous solvents or inert organic solvents. Preferred materials for the subcoat or flow promoting layer include hydroxypropyl cellulose, hydroxyethyl cellulose, hydroxypropyl methyl cellulose, povidone [poly(vinylpyrrolidone)], polyethylene glycol, and mixtures thereof. More preferred are mixtures of hydroxypropyl cellulose and povidone, prepared in organic solvents, particularly organic polar solvents such as lower alkanols having 1-8 carbon atoms, preferable ethanol, mixtures of hydroxyethyl cellulose and hydroxypropyl methyl cellulose prepared in aqueous solution, and mixtures of hydroxyethyl cellulose and polyethylene glycol prepared in aqueous solution. Most preferably, the flow-promoting layer consists of a mixture of hydroxypropyl cellulose and povidone prepared in ethanol.

Conveniently, the weight of the flow-promoting layer applied to the bilayer core may be correlated with the thickness of the flow-promoting layer and residual drug remaining in a dosage form in a release rate assay such as described herein. During manufacturing operations, the thickness of the flow-promoting layer may be controlled by controlling the weight of the subcoat taken up in the coating operation. When the flow-promoting layer is formed as a subcoat, i.e., by coating onto the tableted bilayer composite drug layer and push layer, the subcoat can fill in surface irregularities formed on the bilayer core by the tableting process. The resulting smooth external surface facilitates slippage between the coated bilayer composite and the semipermeable wall during dispensing of the drug, resulting in a lower amount of residual drug composition remaining in the device at the end of the dosing period. When the flow-promoting layer is fabricated of a gel-forming material, contact with water in the environment of use facilitates formation of a gel or gel-like inner coat having a viscosity that may promote and enhance slippage between the semipermeable wall and the drug layer.

The wall is a semipermeable composition, permeable to the passage of an external fluid, such as water and biological fluids, and substantially impermeable to the passage of active agent, osmagent, osmopolymer and the like. The selectively semipermeable compositions used for forming the wall are essentially nonerodible and are insoluble in biological fluids during the life of the dosage form. The wall need not be semipermeable in its entirety, but at least a portion of the wall is semipermeable to allow fluid to contact or communicate with the push displacement layer such that the push layer can imbibe fluid and expand during use. The wall preferably comprises a polymer such as a cellulose acylate, cellulose diacylate, cellulose triacylate, including without limitation, cellulose acetate, cellulose diacetate, cellulose triacetate, or mixtures thereof. The wall forming material may also be selected from ethylene vinyl acetate copolymers, polyethylene, copolymers of ethylene, polyolefins including ethylene oxide copolymers such as Engage^{®} (DuPont Dow Elastomers), polyamides, cellulosic materials, polyurethanes, polyether blocked amides copolymers such as PEBAX^{®} (Elf Atochem North America, Inc.), cellulose acetate butyrate, and polyvinyl acetate. Typically, the wall comprises 60 weight percent (wt %) to 100 wt % of the cellulosic wall-forming polymer, or the wall can comprise 0.01 wt % to 10 wt % of ethylene oxide-propylene oxide block copolymers, known as poloxamers, or 1 wt % to 35 wt % of a cellulose ether selected from the group consisting of hydroxypropylcellulose and hydroxypropylalkylcellulose and 5 wt% to 15 wt% of polyethylene glycol. The total weight percent of all components comprising the wall is equal to 100 wt %.

Representative polymers for forming the wall comprise semipermeable homopolymers, semipermeable copolymers, and the like. Such materials comprise cellulose esters, cellulose ethers and cellulose ester-ethers. The cellulosic polymers have a degree of substitution (DS) of their anhydroglucose unit of from greater than 0 up to 3, inclusive. Degree of substitution (DS) means the average number of hydroxyl groups originally present on the anhydroglucose unit that are replaced by a substituting group or converted into another group. The anhydroglucose unit can be partially or completely substituted with groups such as acyl, alkanoyl, alkenoyl, aroyl, alkyl, alkoxy, halogen, carboalkyl, alkylcarbamate, alkylcarbonate, alkylsulfonate, alkysulfamate, semipermeable polymer forming groups, and the like, wherein the organic moieties contain from one to twelve carbon atoms, and preferably from one to eight carbon atoms.

The semipermeable compositions typically include a cellulose acylate, cellulose diacylate, cellulose triacylate, cellulose acetate, cellulose diacetate, cellulose triacetate, mono-, di- and tri-cellulose alkanylates, mono-, di-, and tri-alkenylates, mono-, di-, and tri-aroylates, and the like. Exemplary polymers include cellulose acetate having a DS of 1.8 to 2.3 and an acetyl content of 32 to 39.9%; cellulose diacetate having a DS of 1 to 2 and an acetyl content of 21 to 35%; cellulose triacetate having a DS of 2 to 3 and an acetyl content of 34 to 44.8%; and the like. More specific cellulosic polymers include cellulose propionate having a DS of 1.8 and a propionyl content of 38.5%; cellulose acetate propionate having an acetyl content of 1.5 to 7% and an acetyl content of 39 to 42%; cellulose acetate propionate having an acetyl content of 2.5 to 3%, an average propionyl content of 39.2 to 45%, and a hydroxyl content of 2.8 to 5.4%; cellulose acetate butyrate having a DS of 1.8, an acetyl content of 13 to 15%, and a butyryl content of 34 to 39%; cellulose acetate butyrate having an acetyl content of 2 to 29%, a butyryl content of 17 to 53%, and a hydroxyl content of 0.5 to 4.7%; cellulose triacylates having a DS of 2.6 to 3, such as cellulose trivalerate, cellulose trilamate, cellulose tripalmitate, cellulose trioctanoate and cellulose tripropionate; cellulose diesters having a DS of 2.2 to 2.6, such as cellulose disuccinate, cellulose dipalmitate, cellulose dioctanoate, cellulose dicaprylate, and the like; and mixed cellulose esters, such as cellulose acetate valerate, cellulose acetate succinate, cellulose propionate succinate, cellulose acetate octanoate, cellulose valerate palmitate, cellulose acetate heptanoate, and the like. Semipermeable polymers are known in U.S. Patent No. 4,077,407, and they can be synthesized by procedures described in Encyclopedia of Polymer Science and Technology, Vol. 3, pp. 325-354, Interscience Publishers Inc., New York, N.Y. (1964).

Additional semipermeable polymers for forming the outer wall comprise cellulose acetaldehyde dimethyl acetate; cellulose acetate ethylcarbamate; cellulose acetate methyl carbamate; cellulose dimethylaminoacetate; semipermeable polyamide; semipermeable polyurethanes; semipermeable sulfonated polystyrenes; cross-linked selectively semipermeable polymers formed by the coprecipitation of an anion and a cation, as disclosed in U.S. Patent Nos. 3,173,876; 3,276,586; 3,541,005; 3,541,006 and 3,546,142; semipermeable polymers, as disclosed by Loeb, et al. in U.S. Patent No. 3,133,132; semipermeable polystyrene derivatives; semipermeable poly(sodium styrenesulfonate); semipermeable poly(vinylbenzyltrimethylammonium chloride); and semipermeable polymers exhibiting a fluid permeability of 10⁻⁵ to 10⁻² (cc. mil/cm hr. atm), expressed as per atmosphere of hydrostatic or osmotic pressure differences across a semipermeable wall. The polymers are known to the art in U.S. Patent Nos. 3,845,770; 3,916,899 and 4,160,020; and in Handbook of Common Polymers, Scott and Roff, Eds., CRC Press, Cleveland, Ohio (1971).

The wall may also comprise a flux-regulating agent. The flux regulating agent is a compound added to assist in regulating the fluid permeability or flux through the wall. The flux-regulating agent can be a flux-enhancing agent or a flux-decreasing agent. The agent can be preselected to increase or decrease the liquid flux. Agents that produce a marked increase in permeability to fluid such as water are often essentially hydrophilic, while those that produce a marked decrease to fluids such as water are essentially hydrophobic. The amount of regulator in the wall when incorporated therein generally is from about 0.01 % to 20% by weight or more. The flux regulator agents may include polyhydric alcohols, polyalkylene glycols, polyalkylenediols, polyesters of alkylene glycols, and the like. Typical flux enhancers include polyethylene glycol 300, 400, 600, 1500, 4000, 6000 and the like; low molecular weight glycols such as polypropylene glycol, polybutylene glycol and polyamylene glycol: the polyalkylenediols such as poly(1,3-propanediol), poly(1,4-butanediol), poly(1,6-hexanediol), and the like; aliphatic diols such as 1,3-butylene glycol, 1,4-pentamethylene glycol, 1,4-hexamethylene glycol, and the like; alkylene triols such as glycerine, 1,2,3-butanetriol, 1,2,4-hexanetriol, 1,3,6-hexanetriol and the like; esters such as ethylene glycol dipropionate, ethylene glycol butyrate, butylene glycol dipropionate, glycerol acetate esters, and the like. Presently preferred flux enhancers include the group of difunctional block-copolymer polyoxyalkylene derivatives of propylene glycol known as poloxamers (BASF). Representative flux-decreasing agents include phthalates substituted with an alkyl or alkoxy or with both an alkyl and alkoxy group such as diethyl phthalate, dimethoxyethyl phthalate, dimethyl phthalate, and [di(2-ethylhexyl) phthalate], aryl phthalates such as triphenyl phthalate, and butyl benzyl phthalate; insoluble salts such as calcium sulfate, barium sulfate, calcium phosphate, and the like; insoluble oxides such as titanium oxide; polymers in powder, granule and like form such as polystyrene, polymethylmethacrylate, polycarbonate, and polysulfone; esters such as citric acid esters esterified with long chain alkyl groups; inert and substantially water impermeable fillers; resins compatible with cellulose based wall forming materials, and the like.

Other materials that may be included in the semipermeable wall material for imparting flexibility and elongation properties to the wall, for malting the wall less brittle to nonbrittle and to render tear strength. Suitable materials include phthalate plasticizers such as dibenzyl phthalate, dihexyl phthalate, butyl octyl phthalate, straight chain phthalates of six to eleven carbons, di-isononyl phthalate, di-isodecyl phthalate, and the like. The plasticizers include nonphthalates such as triacetin, dioctyl azelate, epoxidized tallate, tri-isoctyl trimellitate, tri-isononyl trimellitate, sucrose acetate isobutyrate, epoxidized soybean oil, and the like. The amount of plasticizer in a wall when incorporated therein is about 0.01% to 20% weight, or higher.

### Manufacture of dosage forms

In brief, the dosage forms are manufactured using the following basic steps, which are discussed in greater detail below. The core, which is a bilayer of one drug layer and one push displacement layer, is formed first and coated with the flow-promoting layer; the coated core can then be dried, though this is optional; and the semipermeable wall is then applied. An orifice is then provided by a suitable procedure (e.g., laser drilling), although alternative procedures can be used which provide an orifice which is formed at a later time (a formable orifice). Finally, the finished dosage forms are dried and are ready for use or for coating with an immediate release drug coating.

The drug layer is formed as a mixture containing the active agents (e.g., a nonopioid analgesic and/or opioid analgesic) and the binding agent and other ingredients. The drug layer can be formed from particles by comminution that produces the size of the drug and the size of the accompanying polymer used in the fabrication of the drug layer, typically as a core containing the compound, according to the mode and the manner of the invention. The means for producing particles includes granulation, spray drying, sieving, lyophilization, crushing, grinding, jet milling, micronizing and chopping to produce the intended micron particle size. The process can be performed by size reduction equipment, such as a micropulverizer mill, a fluid energy grinding mill, a grinding mill, a roller mill, a hammer mill, an attrition mill, a chaser mill, a ball mill, a vibrating ball mill, an impact pulverizer mill, a centrifugal pulverizer, a coarse crusher and a fine crusher. The size of the particle can be ascertained by screening, including a grizzly screen, a flat screen, a vibrating screen, a revolving screen, a shaking screen, an oscillating screen and a reciprocating screen. The processes and equipment for preparing the drug and binding agent are disclosed in Pharmaceutical Sciences, Remington, 17th Ed., pp. 1585-1594 (1985); Chemical Engineers Handbook, Perry, 6th Ed., pp. 21-13 to 21-19 (1984); Journal of Pharmaceutical Sciences, Parrot, Vol. 61, No. 6, pp. 813-829 (1974); and Chemical Engineer, Hixon, pp. 94-103 (1990).

Exemplary solvents suitable for manufacturing the respective walls, layers, coatings and subcoatings utilized in the dosage forms of the invention comprise aqueous and inert organic solvents that do not adversely harm the materials utilized to fabricate the dosage forms. The solvents broadly include members selected from the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride nitroethane, nitropropane tetrachloroethane, ethyl ether, isopropyl ether, cyclohexane, cyclooctane, benzene, toluene, naphtha, 1,4-dioxane, tetrahydrofuran, diglyme, water, aqueous solvents containing inorganic salts such as sodium chloride, calcium chloride, and the like, and mixtures thereof such as acetone and water, acetone and methanol, acetone and ethyl alcohol, methylene dichloride and methanol, and ethylene dichloride and methanol.

Pan coating may be conveniently used to provide the completed dosage form, except for the exit orifice. In the pan coating system, the subcoat of the wall-forming compositions can be deposited by successive spraying of the respective composition on the bilayered core comprising the drug layer and the push layer accompanied by tumbling in a rotating pan. A pan coater can be used because of its availability at commercial scale. Other techniques can be used for coating the drug core. The coated dosage form can be dried in a forced-air oven, or in a temperature and humidity controlled oven to free the dosage form of solvent. Drying conditions will be conventionally chosen on the basis of available equipment, ambient conditions, solvents, coatings, coating thickness, and the like.

Other coating techniques can also be employed. For example, the semipermeable wall and the subcoat of the dosage form can be formed in one technique using the air-suspension procedure. This procedure consists of suspending and tumbling the bilayer core in a current of air, an inner subcoat composition and an outer semipermeable wall forming composition, until, in either operation, the subcoat and the outer wall coat is applied to the bilayer core. The air-suspension procedure is well suited for independently forming the wall of the dosage form. The air-suspension procedure is described in U.S. Patent No. 2,799,241; in J. Am. Pharm. Assoc., Vol. 48, pp. 451-459 (1959); and, ibid., Vol. 49, pp. 82-84 (1960). The dosage form also can be coated with a Wurster^{®} air-suspension coater using, for example, methylene dichloride methanol as a cosolvent. An Aeromatic^{®}air-suspension coater can be used employing a cosolvent.

The dosage form of the invention may be manufactured by standard techniques. For example, the dosage form may be manufactured by the wet granulation technique. In the wet granulation technique, the drug and the ingredients comprising the first layer or drug composition are blended using an organic solvent, such as denatured anhydrous ethanol, as the granulation fluid. The ingredients forming the first layer or drug composition are individually passed through a preselected screen and then thoroughly blended in a mixer. Next, other ingredients comprising the first layer can be dissolved in a portion of the granulation fluid, such as the solvent described above. Then, the latter prepared wet blend is slowly added to the drug blend with continual mixing in the blender. The granulating fluid is added until a wet blend is produced, which wet mass blend is then forced through a predetermined screen onto oven trays. The blend is dried for 18 to 24 hours at 24°C to 35°C in a forced-air oven. The dried granules are then sized. Next, magnesium stearate is added to the drug granulation, then put into milling jars and mixed on ajar mill for 10 minutes. The composition is pressed into a layer, for example, in a Manesty^{®}press. The speed of the press is set at 20 rpm and the maximum load set at 2 tons. The first layer is pressed against the composition forming the second layer and the bilayer tablets are fed to the Kilian^{®} Dry Coater press and surrounded with the drug-free coat, followed by the exterior wall solvent coating.

In another manufacture the nonopioid analgesic and opioid analgesic and other ingredients comprising the first layer facing the exit means are blended and pressed into a solid layer. The layer possesses dimensions that correspond to the internal dimensions of the area the layer is to occupy in the dosage form, and it also possesses dimensions corresponding to the second layer for forming a contacting arrangement therewith. The drug and other ingredients can also be blended with a solvent and mixed into a solid or semisolid form by conventional methods, such as ballmilling, calendering, stirring or rollmilling, and then pressed into a preselected shape. Next, the expandable layer, e.g., a layer of osmopolymer composition, is placed in contact with the layer of drug in a like manner. The layering of the drug formulation and the osmopolymer layer can be fabricated by conventional two-layer press techniques. The two contacted layers are first coated with the flow-promoting subcoat and then an outer semipermeable wall. The air-suspension and air-tumbling procedures comprise in suspending and tumbling the pressed, contacting first and second layers in a current of air containing the delayed-forming composition until the first and second layers are surrounded by the wall composition.

Another manufacturing process that can be used for providing the compartment-forming composition comprises blending the powdered ingredients in a fluid bed granulator. After the powdered ingredients are dry blended in the granulator, a granulating fluid, for example, poly(vinylpyrrolidone) in water, is sprayed onto the powders. The coated powders are then dried in the granulator. This process granulates all the ingredients present therein while adding the granulating fluid. After the granules are dried, a lubricant, such as stearic acid or magnesium stearate, is mixed into the granulation using a tote or V-blender. The granules are then pressed in the manner described above.

The flow-promoting layer is then applied to the pressed cores. The semipermeable wall is coated onto the outer surface of the pressed core and/or flow promoting layer. The semi-permeable wall material is dissolved in an appropriate solvent such as acetone or methylene chloride and is then applied to the pressed shape by molding, air spraying, dipping or brushing a solvent-based solution of the wall material onto the shape, as described in U.S. Patent Nos. 4,892,778 and 4,285,987. Other methods for applying the semi-permeable wall include an air suspension procedure, where the pressed shape is suspended and tumbled in a current of air and wall forming material as described in U.S. Patent No. 2,799,241, and a pan coating technique.

After application of the semi-permeable wall to the pressed shape, a drying step is generally required and, then, suitable exit means for the active agent must be formed through the semi-permeable membrane. Depending on the properties of the active agent and other ingredients within the cavity and the desired release rate for the dosage form, one or more orifices for active agent delivery are formed through the semi-permeable membrane by mechanical drilling, laser drilling, or the like.

The exit orifice can be provided during the manufacture of the dosage form or during drug delivery by the dosage form in a fluid environment of use. The expression "exit orifice" as used for the purpose of this invention includes a passageway; an aperture; an orifice; or a bore. The orifice may range in size from a single large orifice encompassing substantially an entire surface of the dosage form to one or more small orifices selectively located on the surface of the semi-permeable membrane. The exit orifice can have any shape, such as round, triangular, square, elliptical and the like for the release of a drug from the dosage form. The dosage form can be constructed with one or more exits in spaced apart relation or one or more surfaces of the dosage form.

The exit orifice may be from 10% to 100% of the inner diameter of the compartment formed by the wall, preferably from 30% to 100%, and most preferably from 50% to 100%. In preferred embodiments, the drug layer is released from the dosage form as an erodible solid through a relatively large orifice of a size of at least 100 mils to 100% of the inner diameter of the compartment formed by the wall, typically from about 125 mils (thousandths of an inch) to about 185 mils, or from about 3.175 to about 4.7 mm. The use of a smaller orifice may be employed if desired to provide a further delay in release of the drug layer.

The exit orifice can be performed by drilling, including mechanical and laser drilling, through the outer coat, the inner coat, or both. Exits and equipment for forming exits are disclosed in, for example, U.S. Patent Nos. 3,845,770 and 3,916,899 to Theeuwes and Higuchi; in U.S. Patent No. 4,063,064 to Saunders, et al.; and in U.S. Patent No. 4,088,864 to Theeuwes, et al.

The exit can also be an orifice that is formed from a substance or polymer that erodes, dissolves or is leached from the outer coat or wall or inner coat to form an exit orifice, as disclosed, for example, in U.S. Patent Nos. 4,200,098 and 4,285,987. Representative materials suitable for forming an orifice, or a multiplicity of orifices comprise leachable compounds, such as a fluid removable pore-former such as inorganic and organic salts, inorganic or organic oxides, carbohydrates, polymers, such as leachable poly(glycolic) acid or poly(lactic) acid polymers, gelatinous filaments, poly(vinyl alcohol), leachable polysaccharides, sugars such as sorbitol, which can be leached from the wall. For example, an exit, or a plurality of exits, can be formed by leaching sorbitol, lactose, fructose, glucose, mannose, galactose, talose, sodium chloride, potassium chloride, sodium citrate and mannitol from the wall.

In addition, in some embodiments, the osmotic dosage form can be in the form of an extruded tube open at one or both ends, as described in commonly owned U.S. Patent No. 6,491,683 to Dong, et al. In the extruded tube embodiment, it is not necessary to provide an additional exit means.

### Pharmaceutically active agents

The sustained release dosage forms provide controlled delivery of pharmaceutically active agents. The sustained release dosage forms are particularly well suited for delivery of insoluble or poorly soluble compounds that are required to be administered in a high dosage to patients.

A wide variety of active agents may be used in the dosage forms. The dosage forms described herein are particularly useful for providing sustained release of difficult to formulate or poorly soluble active agents (e.g., where the solubility of the active agent is less than about 10 mg/ml at 25°C), especially when large doses of these agents are required to be delivered over a prolonged period of time. The dosage forms are also useful for providing sustained release and prolonged delivery of combinations of active agents, and can provide for the proportional delivery of different active agents even when there is a great disparity in solubility between the active agents.

The active agents that can be delivered by the controlled release dosage form comprise inorganic and organic active agents. The active agents include active agents that act on peripheral nerve, adrenergic receptors, cholinergic receptors, the central nervous system, skeletal muscles, the cardiovascular system, smooth muscles, the blood circulatory system, synaptic sites, neuroeffector junctional sites, the endocrine system, hormone systems, the immunological system, organ systems, body passageways, reproductive systems, the skeletal system, autocoid systems, alimentary and excretory systems inhibitors of autocoids and histamine systems, without limitation. The active agents that can be delivered for acting on these recipients include anticonvulsants, analgesics, anti-diabetic agents, anti-parkinson agents, anti-inflammatory agents, anesthetics, antimicrobial agents, antimalarials, antiparasitic agents, antihypertensive agents, angiotensin converting enzyme inhibitors, antihistamines, antipyretics, alpha-adrenergic receptor agonists, alpha-adrenergic receptor blockers, biocides, bactericides, bronchial dilators, beta-adrenergic stimulators, beta-adrenergic blocking drugs, contraceptives, cardiovascular drugs, calcium channel blockers, depressants, diagnostic agents, diuretics, electrolytes, hypnotics, hormonal agents, steroids, antihyperglycemics, muscle contractants, muscle relaxants, ophthalmics, psychic energizers, parasympathomimetics, sedatives, selective androgen receptor modulators, selective estrogen receptor inhibitors, sympathomimetics, tranquilizers, urinary tract drugs, vaginal drugs, and vitamins. Active agents can be included in the sustained release dosage form in free base form, or as a salts, acids, amides, esters, polymorphs, solvates, hydrates, dehydrates, co-crystals, anhydrous, or amorphous forms thereof.

Factors to consider in preparing a particular dosage form are the half life of the drug in the plasma of a patient, the relative bioavailability and absorption of a particular drug in the upper and lower GI tract, whether tolerance develops to a given dose of a drug, whether drug incompatibilities, synergism or interactions occur, the dose required to maintain a particular plasma profile, and the like.

For example, nonsteroidal anti-inflammatory agents or nonopioid analgesics can be delivered using the sustained release dosage forms over a prolonged period of time, enabling a less frequent dosing regimen, such as twice a day dosing, or once a day dosing for active agents having a long half life in plasma. Additional active agents can be included with the nonsteroidal anti-inflammatory agent, for example, for gastric protection. Gastric protective agents include histamine H₂-receptor antagonists (e.g., cimetidine, ranitidine, famotidine, or nizatidine), cytoprotective agents (e.g., misoprostol, rebamipide, ecabet, or carbenoxolone), or proton pump inhibitors (e.g., for example as disclosed in EP-A1-0005129, EP-A1-174 726, EP-A1-166 287, GB 2 163 747 and WO90/06925, WO91/19711, WO91/19712, WO95/01977, WO94/27988, and U.S. Patent No. 6,610,323 to Lundberg, for example, without limitation alpha-pyridylmethylsulfinyl benzimidazoles such as lansoprazole, omeprazole, rabeprazole, pantoprazole, or esomeprazole).

5-HT-agonists can be included in a dosage form delivery NSAIDS for treatment of migrane, for example. 5-HT-agonists include, without limitation, indole derivatives such as triptans, including but not limited to, sumatriptan, eletriptan (described in European Patent Application 379314), Allelix ALX 1323, rizatriptan, frovatriptan, ahnotriptan, zolmitriptan and naratriptan, such as described in U.S. Patent No. 4,816,470; ergot alkaloids such as ergotamine (e.g., ergotamine tartrate), dihydroergotamine, bromocriptine, ergonovine and methyl ergonovine (e.g., ergonovine maleate), methysergide, and ergoloid mesylates, including dihydroergocornine, dihydroergocristine, dihydroergocryptine (alpha and beta), and dihydroergotamine mesylate (DHE 45), and as described in U.S. Patent No. 6,586,458 to Plachetka.

Antibiotics can also be formulated for delivery using the sustained release dosage forms described herein. Any antibiotic that can be administered orally can be included in the controlled release dosage form. Antibiotics include anti-protozoal agents; anti-helminthic agents; agents effective against bacterial species, including gram-positive and gram-negative cocci, gram-positive and gram-negative bacilli, acid-fast bacilli, spirochetes, actinomycetes; species of fungi, such as candida, histoplasma, paracoccidioides, sporothrix, aspergilli, mucormycoses, cryptococci; viruses; as well as miscellaneous organisms such as ureaplasma, mycoplasma, rickettsia, chlamydia, pneumocystis. Exemplary antibiotics include erythromycin, amoxicillin, clarithromycin, tetracycline, or metronidazole. Antibiotics that are poorly soluble, insoluble or poorly dissolving are ideally delivered using the dosage forms described herein. For example, erythromycin is typically required in one or more oral doses of 250 mg (or more) taken four times a day for a total daily dose of 1-2 grams per day. Doses as high as 8 grams per day have been prescribed.

The dosage forms are particularly well suited for the formulation and delivery of poorly soluble compounds such as topiramate, ibuprofen, acetaminophen, erythromycin, gemfibrozil, and the like. The dosage forms can be advantageously used to provide sustained release formulations of nonopioid analgesic agents (particularly acetaminophen) or nonsteroidal anti-inflammatory agents (e.g., ibuprofen, ketoprofen) due to the large doses of these agents needed and the difficulty in formulating and delivering these agents to a patient in need of treatment. In this regard, the combination of opioid analgesics and nonopioid analgesics is a preferred embodiment of dosage forms described herein.

Nonopioid analgesics include the class of compounds known as nonsteroidal anti-inflammatory agents. Examples of nonopioid analgesics include the poorly soluble para-aminophenol derivatives exemplified by acetaminophen, aminobenzoate potassium, aminobenzoate sodium, but can also include nonsteroidal anti-inflammatory agents such as salicylic acid derivatives including aspirin, sulfasalazine, salicylamide, sodium salicylate, and salicylate potassium; aryl propionic acids including benoxaprofen, decibuprofen, flurbiprofen, fenoprofen, ibuprofen, indoprofen, ketoprofen, naproxen, naproxol, oxaprozin; heteroaryl acetic acids such as diclofenac, ketorolac, tolmetin; indole and indene acetic acids including indomethacin, sulindac; selective COX-2 inhibitors such as celecoxib, rofecoxib, valdecoxib, etodolac, ibufenac, nimesulfide, JTE-522, L-745,337, or NS398; alkanones such as nabumetone; oxicams including meloxicam, piroxicam, lornoxicam, cinnoxicam, sudoxicam, tenoxicam; anthranilic acids such as mefenamic acid and meclofenamic acid.
Preferred nonopioid analgesic agents include acetaminophen and ibuprofen. The amount of nonopioid analgesic agent in a single dosage form is typically 0.5 mg to 1000 mg, and more typically between about 200 and about 600 mg.

The active agent can also be an opioid analgesic. Representative opioid analgesics include without limitation alfentanil, allylprodine, alphaprodine, anileridne, benzylmorphine bezitramide, buprenorphine, butorphanol, clonitazene, codeine, cyclazocine, desomorphine, dextromoramide, dezocine, diampromide, dihydrocodeine, dihydromorphine, dimenoxadol, diepheptanol, dimethylthiambutene, dioxaphetyl butyrate, dipipanone, eptazone, ethoheptazine, ethylmethylthiambutene, ethylmorphine, propylmorphine, etonitazene, fentanyl, heroin, hydrocodone, hydromorphone, hydroenitabas, hydrocypethidine, isomethadone, ketobemidone, levallorphan, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphone, phenazocine, phenoperidine, piminodine, pirtramide, propheptazine, promedol, properidine, propiram, propoxyphene, sufentanil, tramadol, and tilidine. The dose of opioid drug 14 is 0.1 µg to 700 mg.

### Methods of use

The dosage forms described above can be used in a variety of methods. For example, the dosage forms can be used in methods for providing an effective concentration of an active agent (e.g., nonopioid analgesic) in the plasma of a human patient for the treatment of a disorder or condition. The dosage forms can also be used in methods for providing sustained release of an active agent (e.g., antibiotics) and delivery to the gastrointestinal tract of a human patient. In particular embodiments, the dosage forms can be used in methods for treating pain in a human patient, for example, in providing an effective amount of an analgesic composition for treating pain, and so forth.

The dosage forms are particularly useful for providing sustained release of poorly soluble or insoluble pharmaceutically active agents, particularly when the active agents are used in combination with additional active agents. The dosage forms provide burst release followed by either an ascending release profile or a zero order release profile. The dosage forms also provide release of the active agents at release rates which are proportional to the respective weights of the active agents in the dosage form, providing a unique ability to tailor the plasma concentration in the patient to either parallel plasma concentrations or differing plasma concentrations, such as would occur if one agent is metabolized at a slower rate than the additional active agent. The active agents can be chosen so that their rates of inactivation or excretion are similar, thus providing a parallel plasma profile, or so that their rates of inactivation or excretion are different, thus providing a plasma profile that diverges.

It is to be understood that while the invention has been described in conjunction with the preferred specific embodiments thereof, that the description above as well as the examples that follow are intended to illustrate and not limit the scope of the invention. The practice of the present invention will employ, unless otherwise indicated, conventional techniques of organic chemistry, polymer chemistry, pharmaceutical formulations, and the like, which are within the skill of the art. Other aspects, advantages and modifications within the scope of the invention will be apparent to those skilled in the art to which the invention pertains. Such techniques are explained fully in the literature.

In the following examples, efforts have been made to ensure accuracy with respect to numbers used (e.g., amounts, temperature, etc.) but some experimental error and deviation should be accounted for. Unless indicated otherwise, temperature is in degrees ° C and pressure is at or near atmospheric. All solvents were purchased as HPLC grade.

Abbreviations:
- HBH:: hydrocodone bitartrate
- HC:: hydrocodone
- HEC:: hydroxyethylcellulose
- HPMC:: hydroxypropylmethylcellulose
- HPC:: hydroxypropylcellulose
- PEO:: poly(ethylene oxide)
- PVP:: polyvinylpyrrolidone

### Example 1

A general procedure for preparing the sustained release dosage forms is as follows:

### Preparation of the Drug Layer Granulation

A binder solution is prepared by adding binding agent (hydroxypropyl cellulose, "HPC" (e.g., Klucel MF, Aqualon Company), or polyvinylpyrrolidone) to water to form a solution containing 5 mg of HPC per 0.995 grams of water. The solution is mixed until the hydroxypropyl cellulose is dissolved. For a particular batch size, a fluid bed granulator ("FBG") bowl is charged with the required amounts of active agent (e.g., ibuprofen at about 80.0% by weight), binding agent (e.g., polyethylene oxide (MW 200,000) (Polyox® N-80, Union Carbide Corporation),, disintegrant (e.g., croscarmellose sodium or crospovidone), optionally surfactant (e.g, polyoxyl 40 stearate or SDS) and osmagent (e.g., sorbitol or mannitol). After mixing the dry materials in the bowl, the binder solution prepared as above is added. Then the granulation is dried in the FBG to a consistency suitable for milling (<1% by weight water), and the granulation is milled through a 7 or a 10 mesh screen.

The granulation is transferred to a tote blender or a V-blender. The required amounts of antioxidant, butylated hydroxytoluene ("BHT") (0.01 %), and lubricant, stearic acid (1%), are sized through a 40 mesh screen and both are blended into the granulation using the tote or V-blender until uniformly dispersed (about 1 minute of blending for stearic acid and about 10 minutes of blending for BHT.

### Preparation of the Osmotic Push Layer Granulation

A binder solution is prepared by adding hydroxypropyl methylcellulose 2910 ("HPMC") to water in a ratio of 5 mg of HPMC to 1 g of water. The solution is mixed until the HPMC is dissolved. Sodium chloride powder (30%) and red ferric oxide (1.0%) are milled and screened. A fluid bed granulator ("FBG") bowl is charged with the required amounts of polyethylene oxide (MW 7,000,000) (Polyox® 303) (63.67%), HPMC (5.0%), the sodium chloride and the red ferric oxide. After mixing the dry materials in the bowl, the binder solution prepared above is added. The granulation is dried in the FBG until the target moisture content (<1% by weight water) is reached. The granulation is milled through a 7 mesh screen and transferred to a tote blender or a V-blender. The required amount of antioxidant, butylated hydroxytoluene (0.08%), is sized through a 60 mesh screen. The required amount of lubricant, stearic acid (0.25%), is sized through a 40 mesh screen and both materials are blended into the granulation using the tote or V-blender until uniformly dispersed (about 1 minute for stearic acid and about 10 minutes for BHT).

### Bilayer Core Compression

A longitudinal tablet press (Korsch press) is set up with round, deep concave punches and dies. Two feed hoppers are placed on the press. The drug layer prepared as above is placed in one of the hoppers while the osmotic push layer prepared as above is placed in the remaining hopper.

The initial adjustment of the tableting parameters (drug layer) is performed to produce cores with a uniform target drug layer weight, typically 300 mg of drug in each tablet. The second layer adjustment (osmotic push layer) of the tableting parameters is performed which bonds the drug layer to the osmotic layer to produce cores with a uniform final core weight, thickness, hardness, and friability. The foregoing parameters can be adjusted by varying the fill space and/or the force setting. A typical tablet containing a target amount of 300 mg of drug will be approximately 0.465 inches long and approximately 0.188 inches in diameter.

### Preparation of the Subcoat Solution and Subcoated System

The subcoat solution is prepared in a covered stainless steel vessel. The appropriate amounts of povidone (K29-32) (2.4%) and hydroxypropyl cellulose (MW 80,000) (Klucel EF, Aqualon Company) (5.6%) are mixed into anhydrous ethyl alcohol (92%) until the resulting solution is clear. The bilayer cores prepared above are placed into a rotating, perforated pan coating unit. The coater is started and after the coating temperature of 28-36° C is attained, the subcoating solution prepared above is uniformly applied to the rotating tablet bed. When a sufficient amount of solution has been applied to provide the desired subcoat weight gain, the subcoat process is stopped. The desired subcoat weight is selected to provide acceptable residuals of drug remaining in the dosage form as determined in the release rate assay for a 24-hour period. Generally, it is desirable to have less than 10%, more preferably less than 5%, and most preferably less than 3% of residual drug remaining after 24 hours of testing in a standard release rate assay as described herein, based on the initial drug loading. This may be determined from the correlation between subcoat weight and the residual drug for a number of dosage forms having the same bilayer core but different subcoat weights in the standard release rate assay.

### Preparation-of the Rate Controlling Membrane and Membrane Coated System

The membrane coating solution contained cellulose acetate 398-10 and poloxamer 188 in varying proportions to obtain a desired water permeation rate into the bilayer cores, and was coated onto the cores to a desired weight gain. Weight gain may be correlated with T₉₀ for membranes of varying thickness in the release rate assay. When a sufficient amount of solution has been applied, conveniently determined by attainment of the desired membrane weight gain for a desired T₉₀, the membrane coating process was stopped.

The coating solution contained 5 wt% solids and was prepared in a 20 gallon closed jacketed stainless steel mixing vessel. The solids (75% cellulose acetate 398-10 and 25% poloxamer 188 or 80% cellulose acetate 398-10 and 20% poloxamer 188, or other desired proportion, both containing trace amounts of BHT, 0.0003%) were dissolved in a solvent that consisted of 99.5% acetone and 0.5% water (w/w) and the appropriate amount of acetone and water were transferred into the mixing vessel. While mixing, the vessel was heated to 25°C to 28°C and then the hot water supply was turned off. The appropriate amount of poloxamer 188, cellulose acetate 398-10 and BHT were charged into the mixing vessel containing the preheated acetone/water solution. The materials were mixed together in the vessel until all the solids were dissolved.

The subcoated bilayer cores (approximately 9 kg per lot) were placed into a Vector Hi-Coater. The coater was started and after the target exhaust temperature was attained, the coating solution was sprayed onto the rotating tablet bed. At regular intervals throughout the coating process, the weight gain was determined. When the desired wet weight gain was achieved, the coating process was stopped.

To obtain coated cores having a particular T₉₀ value, the appropriate coating solution was uniformly applied to the rotating tablet bed until the desired membrane weight gain was obtained. At regular intervals throughout the coating process, the weight gain was determined and sample membrane coated units were tested in the release rate assay as described in Example 4 to determine a T₉₀ for the coated units.

### Drilling of Membrane Coated Systems

One exit port is drilled into the drug layer end of the membrane coated system. During the drilling process, samples are checked at regular intervals for orifice size, location, and number of exit ports.

### Drying of Drilled Coated Systems

Drilled coated systems prepared as above are placed on perforated oven trays which are placed on a rack in a relative humidity oven (43-45% relative humidity) and dried to remove the remaining solvents.

### Color and Clear Overcoats

Optional color or clear coats solutions are prepared in a covered stainless steel vessel. For the color coat 88 parts of purified water is mixed with 12 parts of Opadry II [color not critical] until the solution is homogeneous. For the clear coat 90 parts of purified water is mixed with 10 parts of Opadry Clear until the solution is homogeneous. The dried cores prepared as above are placed into a rotating, perforated pan coating unit. The coater is started and after the coating temperature is attained (35-45.degree. C.), the color coat solution is uniformly applied to the rotating tablet bed. When sufficient amount of solution has been applied, as conveniently determined when the desired color overcoat weight gain has been achieved, the color coat process is stopped. Next, the clear coat solution is uniformly applied to the rotating tablet bed. When sufficient amount of solution has been applied, or the desired clear coat weight gain has been achieved, the clear coat process is stopped. A flow agent (e.g., Car-nu-bo wax) is applied to the tablet bed after clear coat application.

### Example 2

A dosage form containing 350 mg ibuprofen was prepared using the procedures generally described in Example 1. The drug layer composition consisted of the following components: 85 wt % ibuprofen (USP, 38 micron), 6 wt% HPC (NF, Ph Eur), 6 wt% croscarmellose sodium, NF, 2 wt% sodium lauryl sulfate, NF, 0.5 wt% colloidal silicon dioxide, NF, 0.5 wt% magnesium stearate, NF. The push layer contained the following components: 63.67 wt % polyethylene oxide (7000K, NF), 30.0 wt % NaCl, 5 wt% povidone USP, Eur (K29-32), 1 wt% magnesium stearate, NF, Ph Eur, JP, 0.25 wt % ferric oxide, NF, 0.08 wt% BHT, NF. The semipermeable membrane was composed of 80 wt% cellulose acetate, NF (398-10) and 20 wt% poloxamer 188, NF. The orifice size was 155 mils (3.937 mm).

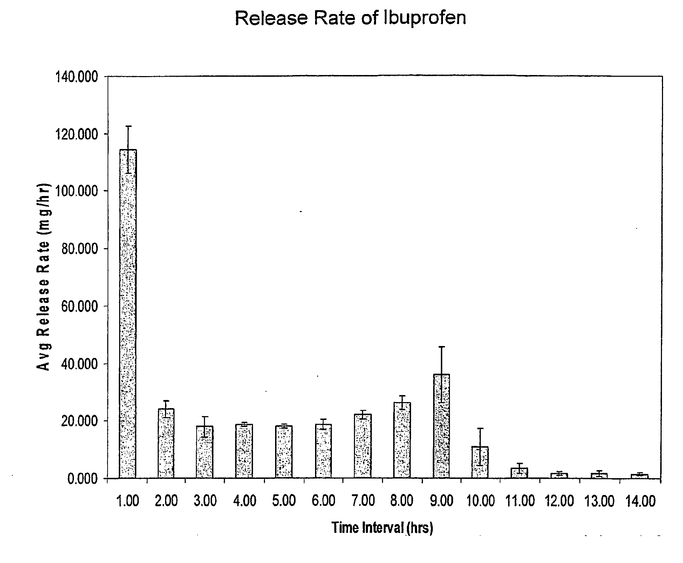

This dosage form produced an initial average rate of release of ibuprofen of 99.5 mg/hr for the first hour, followed by a roughly zero order release rate of about 25 mg/hr sustained for 10 hours, then rapidly dropping off to baseline levels, with a T₉₀ of about 10 hours. Approximately 25% of the dose was delivered at a zero order rate. The results are shown graphically in FIG. 2, and demonstrate the dramatic burst release followed by the sustained release provided by this formulation in the absence of an osmagent.

### Example 3

A dosage form containing 350 mg ibuprofen was prepared using the procedures generally described in Example 1. The drug layer composition consisted of the following components: 85 wt % ibuprofen (USP, 38 micron), 5 wt% HPC (NF, Ph Eur), 3wt% croscarmellose sodium, NF, 3 wt% sodium lauryl sulfate, NF, 3 wt% sorbitol, NF (powder), 0.5 wt% colloidal silicon dioxide, NF, 0.5 wt% magnesium stearate, NF. The push layer contained the following components: 63.67 wt % polyethylene oxide (7000K, NF), 30.0 wt % NaCl, 5 wt% povidone USP, Eur (K29-32), 1 wt% magnesium stearate, NF, Ph Eur, JP, 0.25 wt % ferric oxide, NF, 0.08 wt% BHT, NF. The semipermeable membrane was composed of 80 wt% cellulose acetate, NF (398-10) and 20 wt% poloxamer 188, NF. The orifice size was 155 mils (3.937 mm).

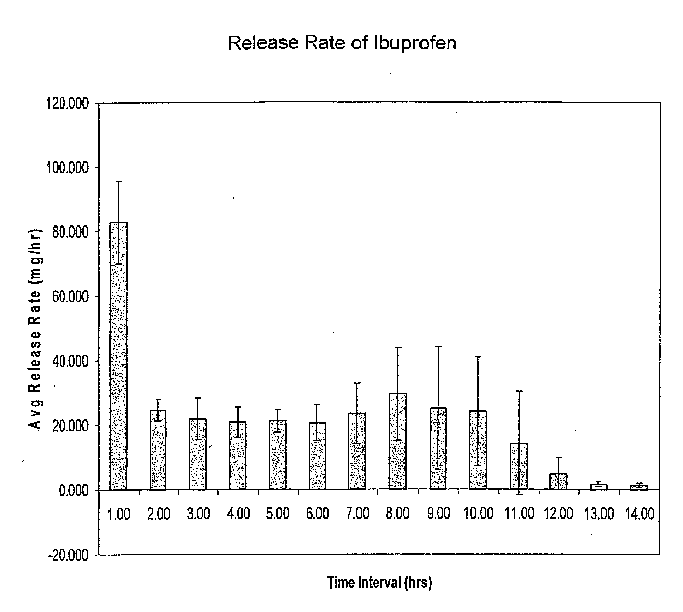

This dosage form produced an initial average rate of release of ibuprofen of 77.7 mg/hr for the first hour, followed by a roughly zero order release rate of about 29 mg/hr sustained for 10 hours, then rapidly dropping off to baseline levels, with a T₉₀ of about 10 hours. Approximately 40% of the dose was delivered at a zero order rate. The results are shown graphically in FIG. 3, and demonstrate the burst release followed by the sustained release provided by this formulation including a small amount of osmagent.

### Example 4

A dosage form containing approximately 350 mg ibuprofen was prepared using the procedures generally described in Example 1. The drug layer composition consisted of the following components: 85 wt % ibuprofen (USP, 38 micron), 7 wt% povidone USP, Ph Eur, (K29-32), 4.0 wt% croscarmellose sodium, NF, 3 wt% sodium lauryl sulfate, NF, 0.5 wt% colloidal silicon dioxide, NF, 0.5 wt% magnesium stearate, NF. The push layer contained the following components: 63.67 wt % polyethylene oxide (7000K, NF), 30.0 wt % NaCl, 5 wt% povidone USP, Eur (K29-32), 1 wt% magnesium stearate, NF, Ph Eur, JP, 0.25 wt % ferric oxide, NF, 0.08 wt% BHT, NF. The semipermeable membrane was composed of 80 wt% cellulose acetate, NF (398-10) and 20 wt% poloxamer 188, NF. The orifice size was 155 mils (3.937 mm).

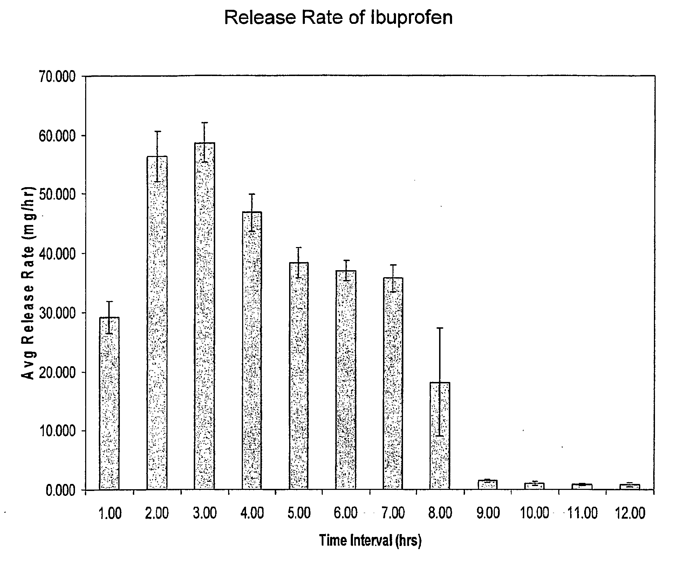

This dosage form produced an initial average rate of release of ibuprofen of 29 mg/hr for the first hour, followed by an increase to a release rate of about 55 mg/hr and a zero order rate of about 40 mg/hr that was sustained for about 4 hours, then rapidly dropped off to baseline levels after 8 hours. The T₉₀ was about 7 hours. The results are shown graphically in FIG. 4, and demonstrate a moderated or delayed burst release and the predominantly zero order sustained release delivery profile provided by this formulation including a fast hydrating binding agent.

### Example 5

A dosage form containing 300 mg ibuprofen was prepared using the procedures generally described in Example 1. The drug layer composition consisted of the following components: 86.0 wt % ibuprofen (USP, 25 micron), 5.0 wt% mannitol, USP (powder), 2.0 wt% HPC, EF, 3.0 wt% croscarmellose sodium, NF, 3.0 wt% sodium lauryl sulfate, NF, 1.0 wt% stearic acid, NF. The push layer contained the following components: 63.67 wt % polyethylene oxide (7000K, NF), 30.0 wt% NaCl, 5 wt% povidone USP, Ph Eur (K29-32), 1 wt% magnesium stearate, NF, Ph Eur, JP, 0.25 wt % ferric oxide, NF, 0.08 wt% BHT, NF. The semipermeable membrane was composed of 75 wt% cellulose acetate, NF (398-10) and 25 wt% poloxamer 188, NF.

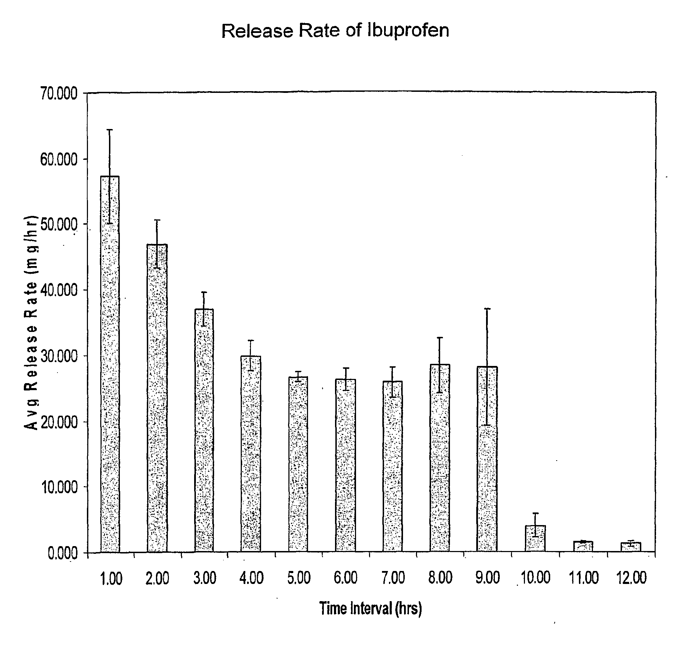

This dosage form produced an initial average rate of release of ibuprofen of 57.3 mg/hr for the first hour, followed by a declining release rate to a zero order release rate of about 30 mg/hr between hours 4 to 9, with a sustained release overall for about 9 hours, before rapidly dropping off to baseline levels, with a T₉₀ of about 8 hours. The results are shown graphically in FIG. 5. These data demonstrate a burst release followed by a sustained zero order delivery profile provided by this formulation containing osmagent.

### Example 6

A dosage form containing 350 mg ibuprofen was prepared using the procedures generally described in Example 1. The drug layer composition consisted of the following components: 80.86 wt % ibuprofen (USP, 25 micron), 4.5 wt% povidone, USP, Ph Eur (K29-32), 4.5 wt% HPC, JF, 4.0 wt% croscarmellose sodium, NF, 3.0 wt% sodium lauryl sulfate, NF, 1.74 wt% hydrocodone bitartrate, 1.0 wt% stearic acid, NF, 0.4 wt% magnesium stearate, NF. The push layer contained the following components: 63.67 wt % polyethylene oxide (7000K, NF), 30.0 wt% NaCl, 5 wt% povidone USP, Ph Eur (K29-32), 1 wt% magnesium stearate, NF, Ph Eur, JP, 0.25 wt % ferric oxide, NF, 0.08 wt% BHT, NF. The semipermeable membrane was composed of 75 wt% cellulose acetate, NF (398-10) and 25 wt% poloxamer 188, NF.

This dosage form produced an initial average rate of release of ibuprofen of 14.5 mg/hr for the first hour, followed by an ascending release rate up to a maximum release rate of about 50 mg/hr at 9 hours, and a sustained release overall for about 9 hours, before rapidly dropping off to baseline levels, with a T₉₀ of about 9 hours. The majority of the dose was delivered at an ascending release rate. The results are shown graphically in FIG. 6, with the release rate data shown in FIG. 6A and the cumulative release in FIG. 6B, demonstrating the complete delivery of the ibuprofen. These data demonstrate the absence of a burst release and the predominant ascending release delivery profile provided by this formulation containing povidone and no osmagent.

### Example 7

A dosage form containing 350 mg ibuprofen was prepared using the procedures generally described in Example 1. The drug layer composition consisted of the following components: 81.85 wt % ibuprofen (USP, 25 micron), 8.0 wt% HPC, NF, 3.0 wt% povidone, USP, Ph Eur (K29-32), 4.0 wt% croscarmellose sodium, NF, 3.0 wt% sodium lauryl sulfate, NF, 1.75 wt% hydrocodone bitartrate, 1.0 wt% stearic acid, NF, 0.4 wt% magnesium stearate, NF. The push layer contained the following components: 63.67 wt % polyethylene oxide (7000K, NF), 30.0 wt% NaCI, 5 wt% povidone USP, Ph Eur (K29-32), 1 wt% magnesium stearate, NF, Ph Eur, JP, 0.25 wt % ferric oxide, NF, 0.08 wt% BHT, NF. The semipermeable membrane was composed of 75 wt% cellulose acetate, NF (398-10) and 25 wt% poloxamer 188, NF.

This dosage form produced an initial average rate of release of ibuprofen of 8.2 mg/hr for the first hour, followed by an ascending release rate up to a maximum release rate of about 67 mg/hr at 8 hours, and a sustained release overall for about 9 hours, before rapidly dropping off to baseline levels, with a T₉₀ of about 9-hours. The majority of the dose was delivered at an ascending release rate. The results are shown graphically in FIG. 7, with the release rate data shown in FIG. 7A and the cumulative release in FIG. 7B. These data demonstrate the absence of a burst release and the predominant ascending release delivery profile provided by this formulation containing a larger proportion of hydroxypropylcellulose and povidone and no osmagent.

### Example 8

A dosage form containing 300 mg ibuprofen was prepared using the procedures generally described in Example 1. The drug layer composition consisted of the following components: 86.0 wt % ibuprofen (USP, 25 micron), 5.0 wt% mannitol, USP (powder), 2.0 wt% HPC, JF, 3.0 wt% croscarmellose sodium, NF, 3.0 wt% sodium lauryl sulfate, NF, 1.0 wt% stearic acid, NF. The push layer contained the following components: 63.67 wt % polyethylene oxide (7000K, NF), 30.0 wt% NaCl, 5 wt% povidone USP, Ph Eur (K29-32), 1 wt% magnesium stearate, NF, Ph Eur, JP, 0.25 wt % ferric oxide, NF, 0.08 wt% BHT, NF. The semipermeable membrane was composed of 75 wt% cellulose acetate, NF (398-10) and 25 wt% poloxamer 188, NF.

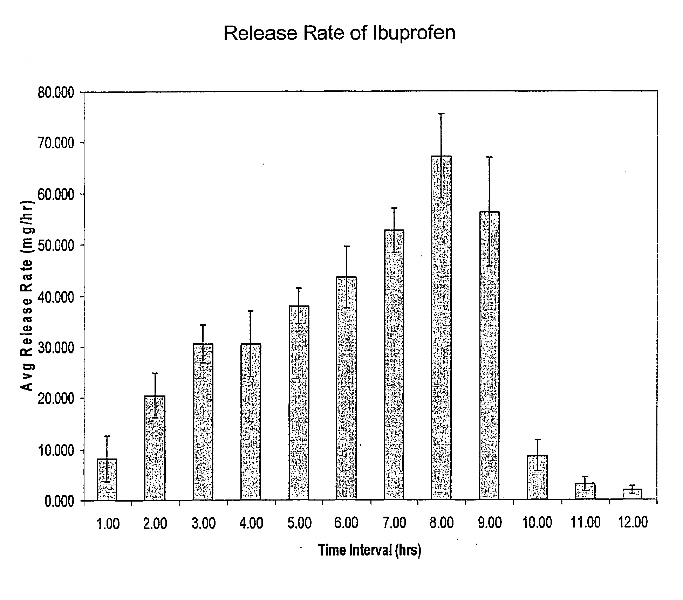

This dosage form produced an initial average rate of release of ibuprofen of 53.3 mg/hr for the first hour, followed by a declining release rate to a minimum rate of 23 mg/hr at about 7 hours, and an ascending release rate to a second maximum release rate of about 38 mg/hr at 9 hours, before rapidly dropping off to baseline levels, with a sustained release overall for about 9 hours and a T₉₀ of about 9 hours. The overall effect resembles a zero order release rate, though the release rate appears to be a balance between an initial burst release and an ascending rate of release. The results are shown graphically in FIG. 8. These data demonstrate a moderate burst release in combination with an ascending rate of release provided by this formulation containing an osmagent and a small amount of binding agent.

### Example 9

A dosage form containing 300 mg ibuprofen was prepared using the procedures generally described in Example 1. The drug layer composition consisted of the following components: 87.0 wt % ibuprofen (USP, 25 micron), 5.0 wt% mannitol, USP (powder), 2.0 wt% HPC, EF, 2.0 wt% croscarmellose sodium, NF, 3.0 wt% sodium lauryl sulfate, NF, 1.0 wt% stearic acid, NF. The push layer contained the following components: 63.67 wt % polyethylene oxide (7000K, NF), 30.0 wt% NaCl, 5 wt% povidone USP, Ph Eur (K29-32), 1 wt% magnesium stearate, NF, Ph Eur, JP, 0.25 wt % ferric oxide, NF, 0.08 wt% BHT, NF. The semipermeable membrane was composed of 75 wt% cellulose acetate, NF (398-10) and 25 wt% poloxamer 188, NF.

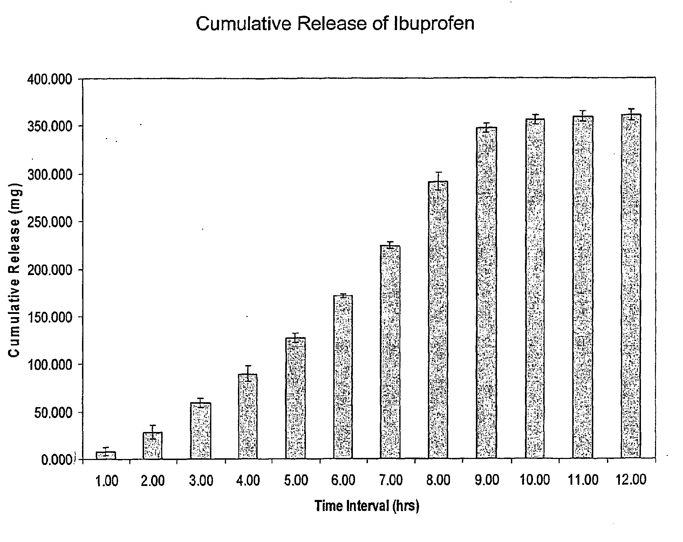

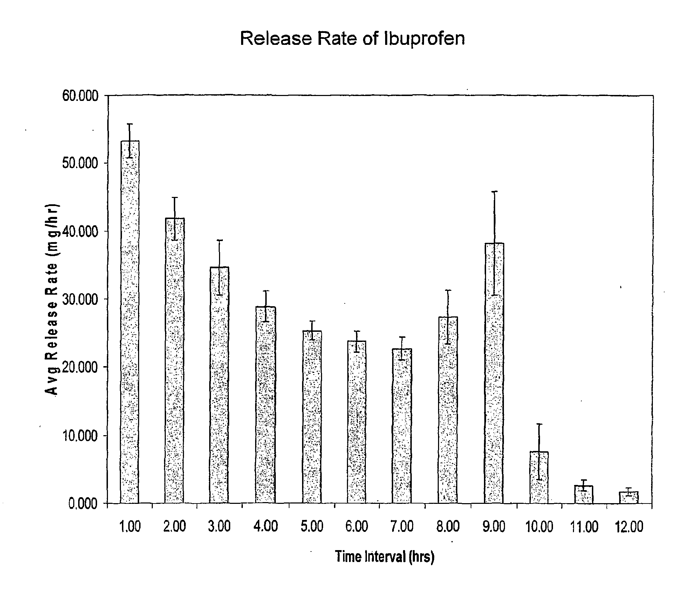

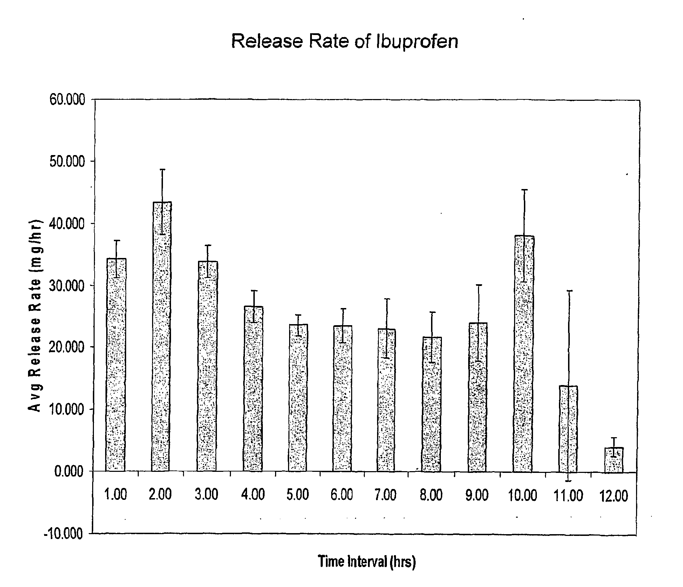

This dosage form produced an initial average rate of release of ibuprofen of 34.2 mg/hr for the first hour, followed by a zero order release rate of about 25-30 mg/hr for 10 hours, with a sustained release overall for about 10 hours, before rapidly dropping off to baseline levels, with a T₉₀ of about 9 hours. The overall effect resembles a zero order release rate. The results are shown graphically in FIG. 9. These data demonstrate a zero order rate of release provided by this formulation containing an osmagent and a relatively small amount of disintegrant and binding agent.

## Claims

1. A sustained release dosage form for delivering a pharmaceutically active agent to a patient in need thereof, comprising:
1) a semipermeable wall defining a cavity and including an exit orifice formed or formable, therein, and
2) an erodible solid contained within the cavity and located adjacent to the exit orifice, said erodible solid comprising a binder, a disintegrant and between 70-90 weight percent of a pharmaceutically active agent,
wherein the erodible solid is released from the dosage form through the exit orifice to provide an immediate release and a sustained release of the pharmaceutically active agent.

2. The sustained release dosage form of claim 1 which provides an ascending rate of release of the active agent.

3. The sustained release dosage form of claim 1 which provides a zero order rate of release of the active agent.

4. The sustained release dosage form of any one of claims 1 to 3, wherein the pharmaceutically active agent has a low solubility in water.

5. The sustained release dosage form of claim 4, wherein the pharmaceutically active agent has a solubility in water of less than 10 mg/ml at 25° C.

6. The sustained release dosage form of any one of claims 1 to 5, wherein the drug loading is from 75% to 85% by weight of the erodible solid.

7. The sustained release dosage form of any preceding claim, wherein the erodible solid further comprises a surfactant and an osmagent.

8. The sustained release dosage form of claim 7, wherein the amount of the pharmaceutically active agent which is immediately released is controlled by the relative proportions of the disintegrant, binding agent, and osmagent and by the solubility of the pharmaceutically active agent.

9. The sustained release dosage form of any preceding claim wherein the pharmaceutically active agent is a nonsteroidal anti-inflammatory agent.

10. The sustained release dosage form of claim 9, wherein the nonsteroidal anti-inflammatory agent is an aryl propionic acid, a COX-2 inhibitor, or ibuprofen.

11. The sustained release dosage form of claim 10, wherein the aryl propionic acid is benoxaprofen, decibuprofen, flurbiprofen, fenoprofen, ibuprofen, indoprofen, ketoprofen, naproxen, naproxol, or oxaprozin, derivatives thereof, or mixtures thereof.

12. The sustained release dosage form of any preceding claim, wherein the binding agent is a hydroxyalkylcellulose, a hydroxyalkylalkylcellulose, or a polyvinylpyrrolidone.

13. The sustained release dosage form of claims 12, wherein the binder is present in the erodible solid at a weight percent of 1 to 10% and wherein the binder is a hydroxyalkylcellulose.

14. The sustained release dosage form of claim 13, wherein the binder is present in the erodible solid at a weight percent of 5% to 6% and wherein the binder is hydroxypropylcellulose.

15. The sustained release dosage form of claim 12, wherein the binder is present in the erodible solid at a weight percent of 1 to 10% and wherein the binder is a polyvinylpyrrolidone

16. The sustained release dosage form of any one of claims 7 to 15, wherein the osmagent is a low molecular weight sugar or a salt.

17. The sustained release dosage form of claim 16, wherein the low molecular weight sugar is sorbitol or mannitol.

18. The sustained release dosage form of any one of claims 7 to 17, wherein the osmagent is present in the erodible solid at a weight percent of from 2% to 10%.

19. The sustained release dosage form of any preceding claim, wherein the disintegrant is present in the erodible solid in an amount of from 1% to 10% by weight.

20. The sustained release dosage form of any preceding claim, wherein the disintegrant is croscarmellose, crospovidone, or sodium alginate.

21. The sustained release dosage form of claim 20, wherein the disintegrant is present in the erodible solid at a weight percent of 2% to 6% and wherein the disintegrant is croscarmellose.

22. The sustained release dosage form of any one of claims 7 to 21, wherein the surfactant is a nonionic or ionic surfactant.

23. The sustained release dosage form of claim 22, wherein the nonionic surfactant is a poloxamer, or a fatty acid ester of polyoxyethylene, or a mixture thereof.

24. The sustained release dosage form of any one of claims 7 to 23, wherein the surfactant is present in the erodible solid at a weight percent of 0.1% to 10%.

25. The sustained release dosage form of claim 24, wherein the surfactant is present in the erodible solid at a weight percent of 2% to 3% and wherein the surfactant is sodium lauryl sulfate.

26. The sustained release dosage form of any one of claims 22 to 25, wherein the surfactant is an ionic surfactant and is an alkali salt of a C₈-C₁₈ alkyl sulfate.

27. The sustained release dosage form of any preceding claim, wherein the dosage form provides a burst release of from 10% to 50% of the pharmaceutically active agent in the first hour after oral administration of the dosage form.

28. The sustained release dosage form of claim 27, wherein the dosage form provides a burst release of from 15% to 30% of the pharmaceutically active agent in the first hour after oral administration of the dosage form.

29. The sustained release dosage form of any one of claims 7 to 28, wherein the rate of release of the pharmaceutically active agent is modulated by the presence of an osmagent in the erodible solid.

30. The sustained release dosage form of any one of claims 7 to 29, wherein the rate of release of the pharmaceutically active agent in the first hour is controlled by the amount of osmagent, binding agent and disintegrant present in the erodible solid.

31. The sustained release dosage form of any one of claims 7 to 30, wherein the rate of release of the pharmaceutically active agent in the first hour is controlled by the relative rates of hydration of the osmagent, binding agent and disintegrant present in the erodible solid.

32. The sustained release dosage form of any one of claims 3 to 31, wherein the dosage form provides a zero order rate of release for at least a portion of the delivery period.

33. The sustained release dosage form of claim 32, wherein the dosage form provides a zero order release from 1 hour to 16 hours after administration.

34. The sustained release dosage form of claim 33, wherein the dosage form provides a zero order release from 1 hour to 10 hours after administration.

35. The sustained release dosage form of any preceding claim, wherein the dosage form releases 90% of the active agent in less than 12 hours.

36. The sustained release dosage form according to any preceding claim, wherein the dosage form provides an ascending rate of release for at least a portion of the delivery period.

37. The sustained release dosage form according to any preceding claim, further comprising:
(1) a push displacement layer contained located distal from the exit orifice; and
(2) a flow-promoting layer between the inner surface of the semipermeable wall and at least the external surface of the drug layer that is opposite the wall.

38. Use of a dosage form according to any preceding claim in the manufacture of a medicament for delivery to a subject for treatment of pain.

## Patentansprüche

1. Eine Darreichungsform mit verzögerter Freigabe zur Verabreichung eines pharmazeutisch wirksamen Mittels an einen Patienten, der dieses benötigt, bestehend aus:
1) einer halbdurchlässigen Wand, die einen Hohlraum definiert sowie einschließlich einer Ausgangsöffnung, die in dieser gebildet ist oder gebildet werden kann; und
2) einem erodierbaren Feststoff, der im Hohlraum enthalten ist und sich neben der Ausgangsöffnung befindet, wobei sich der genannte erodierbare Feststoff aus einem Bindemittel, einem Sprengmittel sowie 70-90 Gewichtprozent eines pharmazeutisch wirksamen Mittels zusammensetzt,
wobei der erodierbare Feststoff von der Darreichungsform durch die Ausgangsöffnung freigesetzt wird, um eine sofortige Freigabe und eine verzögerte Freigabe des pharmazeutischen aktiven Mittels zu liefern.

2. Die Darreichungsform mit verzögerter Freigabe entsprechend Anspruch 1, die eine ansteigende Freisetzungsrate des aktiven Wirkstoffs liefert.

3. Die Darreichungsform mit verzögerter Freigabe entsprechend Anspruch 1, die eine nullte Ordnungsfreisetzungsrate des aktiven Wirkstoffs liefert.

4. Die Darreichungsform mit verzögerter Freigabe entsprechend einem der Ansprüche 1 bis 3, wobei das pharmazeutisch wirksame Mittel eine geringe Wasserlöslichkeit aufweist.

5. Die Darreichungsform mit verzögerter Freigabe entsprechend Anspruch 4, wobei das pharmazeutisch wirksame Mittel eine Wasserlöslichkeit von weniger als 10 mg/l bei 25°C aufweist.

6. Die Darreichungsform mit verzögerter Freigabe entsprechend einem der Ansprüche 1 bis 5, wobei die Wirkstoffbeladung zwischen 75 und 85 Gewichtsprozent des erodierbaren Feststoffs beträgt.

7. Die Darreichungsform mit verzögerter Freigabe entsprechend einem der vorangegangenen Ansprüche, wobei der erodierbare Feststoff zudem ein Tensid und ein osmotische Mittel enthält.

8. Die Darreichungsform mit verzögerter Freigabe entsprechend Anspruch 7, wobei die Menge des pharmazeutisch wirksamen Mittels, die sofort freigegeben wird, durch die relativen Anteile des Sprengmittels, des Bindemittels und des osmotischen Mittels und durch die Löslichkeit des pharmazeutisch wirksamen Mittels kontrolliert wird.

9. Die Darreichungsform mit verzögerter Freigabe entsprechend einem der vorangegangenen Ansprüche, wobei das pharmazeutisch wirksame Mittel ein nicht steroidales entzündungshemmendes Mittel ist.

10. Die Darreichungsform mit verzögerter Freigabe entsprechend Anspruch 9, wobei das nicht steroidale entzündungshemmende Mittel eine Arylpropionsäure, ein COX-2-Hemmer oder Ibuprofen ist.

11. Die Darreichungsform mit verzögerter Freigabe entsprechend Anspruch 10, wobei die Arylpropionsäure Benoxaprofen, Decibuprofen, Flurbiprofen, Fenoprofen, Ibuprofen, Indoprofen, Ketoprofen, Naproxen, Naproxol oder Oxaprozin, ein Derivat dieser oder eine Mischung dieser ist.

12. Die Darreichungsform mit verzögerter Freigabe entsprechend einem der vorangegangenen Ansprüche, wobei das Bindemittel eine Hydroxyalkylcellulose, eine Hydroxyalkylalkylcellulose oder ein Polyvinylpyrrolidon ist.

13. Die Darreichungsform mit verzögerter Freigabe entsprechend Anspruch 12, wobei das Bindemittel im erodierbaren Feststoff zu 1 bis 10 Gewichtsprozent vorhanden ist und wobei das Bindemittel eine Hydroxyalkylcelluslose ist.

14. Die Darreichungsform mit verzögerter Freigabe entsprechend Anspruch 13, wobei das Bindemittel im erodierbaren Feststoff zu 5 bis 6 Gewichtsprozent vorhanden ist und wobei das Bindemittel eine Hydroxypropylcellulose ist.

15. Die Darreichungsform mit verzögerter Freigabe entsprechend Anspruch 12, wobei das Bindemittel im erodierbaren Feststoff zu 1 bis 10 Gewichtsprozent vorhanden ist und wobei das Bindemittel ein Polyvinylpyrrolidon ist.

16. Die Darreichungsform mit verzögerter Freigabe entsprechend einem der Ansprüche 7 bis 15, wobei das osmotische Mittel ein niedermolekularer Zucker oder Salz ist.

17. Die Darreichungsform mit verzögerter Freigabe entsprechend Anspruch 16, wobei der niedermolekulare Zucker Sorbitol oder Mannitol ist.

18. Die Darreichungsform mit verzögerter Freigabe entsprechend einem der Ansprüche 7 bis 17, wobei das osmotische Mittel im erodierbaren Feststoff zu 2 bis 10 Gewichtsprozent vorhanden ist.

19. Die Darreichungsform mit verzögerter Freigabe entsprechend einem der vorangegangenen Ansprüche, wobei das Sprengmittel im erodierbaren Feststoff zu 1 bis Gewichtsprozent vorhanden ist.

20. Die Darreichungsform mit verzögerter Freigabe entsprechend einem der vorangegangenen Ansprüche, wobei das Sprengmittel Croscarmellose, Crospovidon oder Natriumalginat ist.

21. Die Darreichungsform mit verzögerter Freigabe entsprechend Anspruch 20, wobei das Sprengmittel im erodierbaren Feststoff zu 2 bis 6 Gewichtsprozent vorhanden ist und wobei das Sprengmittel Croscarmellose ist.

22. Die Darreichungsform mit verzögerter Freigabe entsprechend einem der Ansprüche 7 bis 21, wobei das Tensid ein nichtionisches oder ionisches Tensid ist.

23. Die Darreichungsform mit verzögerter Freigabe entsprechend Anspruch 22, wobei das nichtionische Tensid eine Poloxamer oder ein Fettsäureester von Polyoxyethylen oder eine Mischung derer ist.

24. Die Darreichungsform mit verzögerter Freigabe entsprechend einem der Ansprüche 7 bis 23, wobei das Tensid im erodierbaren Feststoff zu 0,1 bis 10 Gewichtsprozent vorhanden ist.

25. Die Darreichungsform mit verzögerter Freigabe entsprechend Anspruch 24, wobei das Tensid im erodierbaren Feststoff zu 2 bis 3 Gewichtsprozent vorhanden ist und wobei das Tensid Natriumlaurylsulfat ist.

26. Die Darreichungsform mit verzögerter Freigabe entsprechend einem der Ansprüche 22 bis 25, wobei das Tensid ein ionisches Tensid und Alkalisalz von C₈-C₁₈-Alkylsulfat ist.

27. Die Darreichungsform mit verzögerter Freigabe entsprechend einem der vorangegangenen Ansprüche, wobei die Darreichungsform in der ersten Stunde nach oraler Verabreichung der Darreichungsform eine Impulsfreisetzung von 10% bis 50% des pharmazeutisch wirksamen Mittels liefert.

28. Die Darreichungsform mit verzögerter Freigabe entsprechend Anspruch 27, wobei die Darreichungsform in der ersten Stunde nach oraler Verabreichung der Darreichungsform eine Impulsfreisetzung von 15% bis 30% des pharmazeutisch wirksamen Mittels liefert.

29. Die Darreichungsform mit verzögerter Freigabe entsprechend einem der Ansprüche 7 bis 28, wobei die Freisetzungsrate des pharmazeutisch wirksamen Mittels durch die Gegenwart eines osmotischen Mittels im erodierbaren Feststoff moduliert wird.

30. Die Darreichungsform mit verzögerter Freigabe entsprechend einem der Ansprüche 7 bis 30, wobei die Freisetzungsrate des pharmazeutisch wirksamen Mittels während der ersten Stunde durch die Menge an osmotischem Mittel, Bindemittel und Sprengmittel, die im erodierbaren Feststoff vorhanden sind, kontrolliert wird.

31. Die Darreichungsform mit verzögerter Freigabe entsprechend einem der Ansprüche 7 bis 30, wobei die Freisetzungsrate des pharmazeutisch wirksamen Mittels während der ersten Stunde durch die relativen Hydrationsraten des osmotischen Mitteln, Bindemitteln und Sprengmitteln, die im erodierbaren Feststoff vorhanden sind, kontrolliert wird.

32. Die Darreichungsform mit verzögerter Freigabe entsprechend einem der Ansprüche 3 bis 30, wobei die Darreichungsform eine nullte Ordnungsfreisetzungsrate für mindestens eines Teil des Abgabezeitraums liefert.

33. Die Darreichungsform mit verzögerter Freigabe entsprechend Anspruch 32, wobei die Darreichungsform eine nullte Ordnungsfreisetzungsrate von 1 Stunde bis 16 Stunden nach der Verabreichung liefert.

34. Die Darreichungsform mit verzögerter Freigabe entsprechend Anspruch 33, wobei die Darreichungsform eine nullte Ordnungsfreisetzungsrate von 1 Stunde bis 10 Stunden nach der Verabreichung liefert.

35. Die Darreichungsform mit verzögerter Freigabe entsprechend einem der vorangegangenen Ansprüche, wobei die Darreichungsform in weniger als 12 Stunden 90% des aktiven Wirkstoffs freigibt.

36. Die Darreichungsform mit verzögerter Freigabe entsprechend einem der vorangegangenen Ansprüche, wobei die Darreichungsform für zumindest einen Teil des Abgabezeitraums eine ansteigende Freisetzungsrate liefert.

37. Die Darreichungsform mit verzögerter Freigabe entsprechend einem der vorangegangenen Ansprüche, zu der noch folgende gehören:
(1) einer verschiebbaren Schicht, die im sich distal von der Ausgangsöffnung befindet, und
(2) einer fließfördernden Schicht zwischen der Innenfläche der halbdurchlässigen wand und mindestens einer Außenfläche der Medikamentenschicht, die gegenüber der Wand ist.

38. Verwendung einer Darreichungsform entsprechend einem der vorangegangenen Ansprüche bei der Herstellung eines Arzneimittels zur Verabreichung an einen Patienten zur Schmerzbehandlung.

## Revendications

1. Forme dosifiée à libération prolongée permettant l'administration d'un agent actif à un patient en ayant besoin, comprenant :
1) une paroi semi-perméable définissant une cavité et comprenant un orifice de sortie formé ou formable dans la cavité, et
2) un solide érodable contenu dans la cavité et monté adjacent à l'orifice de sortie, ledit solide érodable comprenant un liant, un désintégrant et entre 70 à 90 pourcent en poids d'un agent pharmaceutiquement actif,
dans lequel le solide érodable est libéré de la forme dosifiée par l'orifice de sortie afin de procurer une libération immédiate et une libération prolongée de l'agent pharmaceutiquement actif.

2. Forme dosifiée à libération prolongée de la revendication 1 qui procure un taux ascendant de l'agent actif.

3. Forme dosifiée à libération prolongée de la revendication 1 qui procure un taux de libération de l'agent actif de l'ordre de zéro.

4. Forme dosifiée à libération prolongée de l'une quelconque des revendications 1 à 3, dans laquelle l'agent pharmaceutiquement actif présente une faible solubilité dans l'eau.

5. Forme dosifiée à libération prolongée de la revendication 4, dans laquelle l'agent pharmaceutiquement actif présente une solubilité dans l'eau de pas moins de 10 mg/ml à 25°C.

6. Forme dosifiée à libération prolongée de l'une quelconque des revendications 1 à 5, dans laquelle la charge médicamenteuse représente 75% à 85% en poids celle du solide érodable.

7. Forme dosifiée à libération prolongée d'une quelconque revendication précédente, dans laquelle le solide érodable contient en outre un surfactant et un agent osmotique.

8. Forme dosifiée à libération prolongée de la revendication 7, dans laquelle la quantité de l'agent pharmaceutiquement actif qui est libérée immédiatement est déterminée par les proportions relatives du désintégrant, de l'agent liant et de l'agent osmotique et par la solubilité de l'agent pharmaceutiquement actif.

9. Forme dosifiée à libération prolongée d'une quelconque revendication précédente dans laquelle l'agent pharmaceutiquement actif est un agent anti-inflammatoire non stéroïdien.

10. Forme dosifiée à libération prolongée de la revendication 9, dans laquelle l'agent anti-inflammatoire non stéroïdien est un acide arylpropionique, un inhibiteur des COX-2 ou l'ibuprofène.

11. Forme dosifiée à libération prolongée de la revendication 10, dans laquelle l'acide arylpropionique est le bénoxaprofène, le décibuprofène, le flurbiprofène, le fénoprofène, l'ibuprofène, l'indoprofène, le kétoprofène, le naproxène, le naproxol ou l'oxaprozine, des dérivés ou des mélanges de ceux-ci.

12. Forme dosifiée à libération prolongée d'une quelconque revendication précédente, dans laquelle l'agent liant est une hydroxyalkylcellulose, une hydroxyalkylalkylcellulose ou un polyvinylpyrrolidone.

13. Forme dosifiée à libération prolongée des revendications 12, dans laquelle le liant est présent dans le solide érodable à un pourcentage en poids de 1 à 10% et dans laquelle le liant est une hydroxyalkylcellulose.

14. Forme dosifiée à libération prolongée de la revendication 13, dans laquelle le liant est présent dans le solide érodable à un pourcentage en poids de 5% à 6% et dans laquelle le liant est de l'hydroxypropylcellulose.

15. Forme dosifiée à libération prolongée de la revendication 12, dans laquelle le liant est présent dans le solide érodable à un pourcentage en poids de 1 à 10% et dans laquelle le liant est un polyvinylpyrrolidone.

16. Forme dosifiée à libération prolongée de l'une quelconque des revendications 7 à 15, dans laquelle l'agent osmotique est un sel ou un sucre à faible poids moléculaire.

17. Forme dosifiée à libération prolongée de la revendication 16, dans laquelle le sucre à faible poids moléculaire est du sorbitol ou du mannitol.

18. Forme dosifiée à libération prolongée de l'une quelconque des revendications 7 à 17, dans laquelle l'agent osmotique est présent dans le solide érodable à un pourcentage en poids allant de 2% à 10%.

19. Forme dosifiée à libération prolongée d'une quelconque revendication précédente, dans laquelle le désintégrant est présent dans le solide érodable selon une quantité de 1% à 10% en poids.

20. Forme dosifiée à libération prolongée d'une quelconque revendication précédente, dans laquelle le désintégrant est de la croscarmellose, du crospovidone ou de l'alginate de sodium.

21. Forme dosifiée à libération prolongée de la revendication 20, dans laquelle le désintégrant est présent dans le solide érodable à un pourcentage en poids de 2 à 6% et dans laquelle le désintégrant est de la croscarmellose.

22. Forme dosifiée à libération prolongée de l'une quelconque des revendications 1 à 21, dans laquelle le surfactant est un surfactant non ionique ou ionique.

23. Forme dosifiée à libération prolongée de la revendication 22, dans laquelle le surfactant non ionique est un poloxamère, ou un ester d'acide gras de polyoxyéthylène ou un mélange de ceux-ci.

24. Forme dosifiée à libération prolongée de l'une quelconque des revendications 7 à 23, dans laquelle le surfactant est présent dans le solide érodable à un pourcentage en poids de 0,1% à 10%.

25. Forme dosifiée à libération prolongée de la revendication 24, dans laquelle le surfactant est présent dans le solide érodable à un pourcentage en poids de 2 à 3% et dans laquelle le surfactant est du laurylsulfate de sodium.

26. Forme dosifiée à libération prolongée de l'une quelconque des revendications 22 à 25, dans laquelle le surfactant est un surfactant ionique et est un sel alcalin du sulfate d'alkyle C₈-C₁₈.

27. Forme dosifiée à libération prolongée d'une quelconque revendication précédente, dans laquelle la forme dosifiée procure une libération en pics de 10% à 50% de l'agent pharmaceutiquement actif durant la première heure suivant l'administration par voie orale de la forme dosifiée.

28. Forme dosifiée à libération prolongée de la revendication 27, dans laquelle la forme dosifiée procure une libération en pics de 15% à 30% de l'agent pharmaceutiquement actif durant la première heure suivant l'administration par voie orale de la forme dosifiée.

29. Forme dosifiée à libération prolongée selon l'une des revendications 7 à 28, dans laquelle le taux de libération de l'agent pharmaceutiquement actif est modulé par la présence d'un agent osmotique dans le solide érodable.

30. Forme dosifiée à libération prolongée de l'une quelconque des revendications 7 à 29, dans laquelle le taux de libération de l'agent pharmaceutiquement actif durant la première heure est déterminé par la quantité d'agent osmotique, d'agent liant et de désintégrant présente dans le solide érodable.

31. Forme dosifiée à libération prolongée de l'une quelconque des revendications 7 à 30, dans laquelle le taux de libération de l'agent pharmaceutiquement actif durant la première heure est déterminé par les taux relatifs de l'agent osmotique, de l'agent liant et du désintégrant présents dans l'agent érodable.

32. Forme dosifiée à libération prolongée de l'une quelconque des revendications 3 à 31, dans laquelle la forme dosifiée procure un taux de libération de l'ordre de zéro pour au moins une partie de la période de libération.

33. Forme dosifiée à libération prolongée de la revendication 32, dans laquelle la forme dosifiée procure une libération de l'ordre de zéro 1 heure à 16 heures après l'administration.

34. Forme dosifiée à libération prolongée de la revendication 13, dans laquelle la forme dosifiée procure une libération de l'ordre de zéro 1 heure à 10 heures après l'administration.

35. Forme dosifiée à libération prolongée selon une quelconque revendication précédente, dans laquelle la forme dosifiée libère 90% de l'agent actif en moins de 12 heures.

36. Forme dosifiée à libération prolongée selon une quelconque revendication précédente, dans laquelle la forme dosifiée procure un taux de libération ascendant pour au moins une partie de la période de libération.

37. Forme dosifiée à libération prolongée selon une quelconque revendication précédente, comprenant en outre :
(1) une couche de déplacement par expulsion confinée située à un emplacement distal de l'orifice de sortie ; et
(2) une couche favorisant l'écoulement entre la surface intérieure de la paroi semi-perméable et au moins la surface extérieure de la couche médicamenteuse qui est opposée à la paroi.

38. Utilisation de la forme dosifiée selon une quelconque revendication précédente dans la fabrication d'un médicament en vue de son administration à un sujet pour le traitement de la douleur.
